# EUROPÄISCHE PATENTSCHRIFT

(11) **EP 3 130 399 B1**
(45) Veröffentlichungstag und Bekanntmachung des Hinweises auf die Patenterteilung: **07.07.2021**
(21) Anmeldenummer: 16183758.8
(22) Anmeldetag: 11.08.2016
(51) Int. Cl.: B01J 27/185, B01J 37/18, B01J 37/02, C07C 45/50, B01J 21/08, B01J 21/18, B01J 27/224

(54) **VERFAHREN ZUR HYDROFORMYLIERUNG VON OLEFINEN UND/ODER ALKINEN IN DER GASPHASE MIT EINEM GEMISCH AUS WASSERSTOFF UND KOHLENMONOXID IN GEGENWART VON HETEROGENEM KATALYSATOR**
METHOD FOR THE HYDROFORMYLATION OF OLEFINS AND/OR ALKYNES IN THE GAS PHASE WITH A MIXTURE OF HYDROGEN AND CARBON MONOXIDE IN THE PRESENCE OF HETEROGENEOUS CATALYST
PROCEDE D'HYDROFORMYLATION D'OLEFINES ET/OU D'ALCYNES EN PHASE GAZEUSE A L'AIDE D'UN MELANGE D'HYDROGENE ET DE MONOXYDE DE CARBONE EN PRESENCE DE CATALYSEUR HETEROGENE

(30) Priorität: 12.08.2015 EP 15180813
(43) Veröffentlichungstag der Anmeldung: 15.02.2017
(73) Patentinhaber: BASF SE, 67056 Ludwigshafen am Rhein (DE)
(72) Erfinder: Schunk, Stephan A, 69126 Heidelberg-Rohrbach (DE); Lejkowski, Michael, 69151 Neckargemuend (DE); Titlbach, Sven, 69115 Heidelberg (DE); Alvarado Rupflin, Luis Tojil, 68163 Mannheim (DE); Kaiser, Harry, 69181 Leimen (DE)
(74) Vertreter: BASF IP Association

(56) Entgegenhaltungen:
- CH-A- 314 911
- US-A1- 2014 018 455
- Bravo-Suárez, Juan J. et al.: "Design of Heterogeneous Catalysts for Fuelsand Chemicals Processing: An Overview" In: Bravo-Suárez, Juan J. et al.: "Novel Materials for Catalysis and Fuels Processing (ACS Symposium Series)", 11. Juni 2013 (2013-06-11), ACS, Washington, DC, XP002753837, Bd. 1132, Seiten 3-68, DOI: 10.1021/bk-2013-1132.ch001, * Ref [127], [130]; Seite 20 - Seite 21 *
- OYAMA T: "2.3.14 Transition Metal Carbides, Nitrides, and Phosphides"; "2. PREPARATION OF SOLID CATALYSTS" In: Ertl G et al. (eds): "HANDBOOK OF HETEROGENEOUS CATALYSIS", 15. März 2008 (2008-03-15), WILEY-VCH, PAGE(S) 342 - 356, Weinheim (DE), XP009122791, ISBN: 978-3-527-31241-2 Bd. 1, Seiten 342-356, DOI: 10.1002/9783527610044.hetcat0019, * das ganze Dokument *
- Loretta Storaro ET AL: "JOURNAL OF MOLECULAR CATALYSIS Highly selective vapor phase propene hydroformylation catalyzed by Rh/B and Rh-Co/B systems on silica", Journal of Molecular Catalysis A: Chemical, Bd. 112, Nr. 1 1. Januar 1996 (1996-01-01), Seiten 43-54, XP055247027, Gefunden im Internet: URL:http://ac.els-cdn.com/1381116996002439 /1-s2.0-1381116996002439-main.pdf?_tid=a28 239d8-c9ea-11e5-874a-00000aab0f01&acdnat=1 454444709_25399c44e4180cd3bdc2c8b3514f9a4b
- ROEL PRINS ET AL: "Metal Phosphides: Preparation, Characterization and Catalytic Reactivity", CATALYSIS LETTERS, KLUWER ACADEMIC PUBLISHERS-PLENUM PUBLISHERS, NE, Bd. 142, Nr. 12, 9. November 2012 (2012-11-09), Seiten 1413-1436, XP035145939, ISSN: 1572-879X, DOI: 10.1007/S10562-012-0929-7
- STEPHANIE L. BROCK ET AL: "Chemical Routes for Production of Transition-Metal Phosphides on the Nanoscale: Implications for Advanced Magnetic and Catalytic Materials", CHEMISTRY - A EUROPEAN JOURNAL., Bd. 10, Nr. 14, 9. März 2004 (2004-03-09), Seiten 3364-3371, XP055246773, WEINHEIM, DE ISSN: 0947-6539, DOI: 10.1002/chem.200305775
- AYAKA SAWADA ET AL: "Rhodium phosphide catalyst for hydrodesulfurization: Low temperature synthesis by sodium addition", CATALYSIS COMMUNICATIONS, Bd. 56, 6. Juli 2014 (2014-07-06), Seiten 60-64, XP055246761, NL ISSN: 1566-7367, DOI: 10.1016/j.catcom.2014.06.027
- SCHULZ H ED - HISATOMI TAKASHI ET AL: "Short history and present trends of Fischer-Tropsch synthesis", APPLIED CATALYSIS A: GENERAL, ELSEVIER, AMSTERDAM, NL, Bd. 186, Nr. 1-2, 4. Oktober 1999 (1999-10-04), Seiten 3-12, XP004271921, ISSN: 0926-860X, DOI: 10.1016/S0926-860X(99)00160-X
- QIAN LIU ET AL: "Carbon Nanotubes Decorated with CoP Nanocrystals: A Highly Active Non-Noble-Metal Nanohybrid Electrocatalyst for Hydrogen Evolution", ANGEWANDTE CHEMIE INTERNATIONAL EDITION, Bd. 53, Nr. 26, 20. Mai 2014 (2014-05-20), Seiten 6710-6714, XP055329648, DE ISSN: 1433-7851, DOI: 10.1002/anie.201404161 -& Qian Liu ET AL: "Supporting Information: Carbon Nanotubes Decorated with CoP Nanocrystals: A Highly Active Non-Noble-Metal Nanohybrid Electrocatalyst for Hydrogen Evolution", Angewandte Chemie International Edition, 20. Mai 2014 (2014-05-20), Seiten S1-S16, XP055329721, DOI: 10.1002/anie.201404161 Gefunden im Internet: URL:http://onlinelibrary.wiley.com/store/1 0.1002/anie.201404161/asset/supinfo/anie_2 01404161_sm_miscellaneous_information.pdf? v=1&s=ae8ccf33915de86983d73beec6fe11ab4f66 c635 [gefunden am 2016-12-15]
- None

## Beschreibung

Die Erfindung betrifft einen heterogenen Katalysator mit einer katalytisch aktiven Komponente, die als katalytisch aktive Komponente Phosphid umfasst und ein Verfahren zur Herstellung von C₃- bis C₁₉-Aldehyden und/oder Alkoholen durch die Hydroformylierung von Olefinen und/oder Alkinen in der Gasphase mit einem Gemisch aus Wasserstoff und Kohlenmonoxid. Die Umsetzung erfolgt bei erhöhtem Druck und erhöhter Temperatur, in An- oder Abwesenheit eines Trägers und/oder von Promotoren. Als katalytisch aktive Komponente wird das Phosphid eines oder mehrerer Metalle, ausgewählt aus der Gruppe Co, Rh verwendet.

Im Stand der Technik wird von Lenarda et al. (im Journal of Molecular Catalysis, 80 (1993) S. 105 - 116) ein Verfahren zur Hydroformylierung von Propen in der Gasphase in Gegenwart eines Katalysators offenbart, wobei die Reaktion durch ein System von Rh/Al auf Silica katalysiert wird. Bekannt ist auch, dass im Katalysator das Aluminium durch Bor ersetzt werden kann.

In der Veröffentlichung von L. Storaro sowie Lenarda et al. wird (im Journal of Molecular Catalysis 112 (1996) S. 43 - 54) katalytische Hydrofomylierung von Propen in der Gasphase beschrieben. Die von Storaro et al. verwendeten Katalysator enthalten Rh/B auf Silica und Rh-Co/B auf Silica.

In der Veröffentlichung von Bo Wang et al. wird (in Appl. Nanosci. (2012) 2: 423 - 427; DOI 10.1007/s13204-012-0057-0) eine Hydrothermalsynthese zur Herstellung von Katalysatoren mit nanopartikulärem Nickelphosphid beschrieben.

In der US 8,586,800 B2 offenbart Richard W. Wegmann die einen Prozess zur Hydroformylierung in der Gasphase zur Herstellung von zumindest einem Aldehyd. Die Anmelderin der US 8,586,800 B2, die am 21.04.2011 als PCT-Anmeldung publiziert wurde, ist die Firma Dow Technology. Der Prozess erfolgt in der Gegenwart eines Katalysators mit Metall-Ligandenkomplex und in Anwesenheit von Wasser. Auch bei sehr geringem Gehalt an Wasserdampf im Gasstrom kann die Aktivität des Katalysators aufrechterhalten werden.

Die Verwendung von Katalysatoren mit Rhodiumphosphid als Aktivkomponente zur Hydroentschwefelung wird in einer Veröffentlichung von Ayaka Sawada et al. (in Catalysis Communication 56 (2014) S. 60 - 64) beschrieben. In der Veröffentlichung Sawada et al. wird Effekt der Natriumzugabe auf die Bildung von Rhodiumphosphid auf MFI Zeolith, auf SiO₂ und auf Al₂O₃ sowie die Aktivität bei der Hydroentschwefelung untersucht.

Chemische Routen zur Herstellung von Phosphiden der Übergangsmetalle, die in nanoskaliger Form vorliegen, werden in der Veröffentlichung von Stephanie L. Brock et al. (in Chemistry Europe Journal, 2004, 10, S. 3364 - 3371) beschrieben. Es wird dargelegt, dass die Materialien sich als magnetische Materialien und als katalytische Materialien eignen. Das Interesse an geträgerten Phosphiden der Übergangsmetalle ist durch deren Funktion als heterogene Katalysatoren angetrieben worden. Die Aufmerksamkeit galt der Aktivität der Katalysatoren im Bereich Hydrotreatment. Bezüglich der Hydroentschwefelung ist auch auf die Veröffentlichung von S. Ted Oyama (in Journal of Catalysis 216 (2003) S. 343 - 352 zu verweisen, in der Übergangsmetallphosphide als neue Katalysatoren für fortschrittliches Hydro-Processing beschrieben werden. Als Träger für die Übergangsmetallphosphide wird SiO₂ oder Al₂O₃ eingesetzt.

Weiterhin gibt es von Ted Oyama ein Übersichtskapitel zum Thema Übergangsmetallcarbide, Nitride und Phosphide ist im Handbuch für heterogene Katalyse Transition (siehe Metal Carbides, Nitrides, and Phosphides in Ertl G et al. (eds): "HANDBOOK OF HETEROGENEOUS CATALYSIS" 15. März 2008, (200815), WILEY-VCH, PAGES(S) 352-356, Weinheim (DE), XP009122791, ISBN: 978-3-527-31241-2; Bd. 1, Seiten 352-356, DOI: 10.1002/9783527610044.hetcat0019). Es wird berichtet, dass die Übergangsmetallphosphide mit Ni, Fe oder Mo in HDS-Reaktionen (d.h. zur Katalyse von Hydrogen-Desulfurisierungs-Prozessen) eingesetzt werden. Es wird darüber berichtet, dass auch Co2P Aktivität in HDS- und HDN-Reaktionen aufweist.

Von Juan J. Bravo-Suärez et al. wird (ACS Symposium Series, 2013, Washington DC, XP002753837, Bd. 1132, S. 3-68, DOI: 10.1021/bk-2013-1132.ch001 ein Übersichtsartikel zum Design von heterogenen Katalysatoren zur Prozessierung von Treibstoffen und Chemikalien gegeben. Der Übersichtsartikel enthält auf den Seiten 20 - 21 eine Darstellung zu Übergangsmetallphosphiden, Metallcarbiden und Nitriden. Als die herausstechenden Merkmale dieser Materialien werden die platinähnlichen Eigenschaften von Metallcarbiden und Nitriden und außergewöhnliche Eigenschaften von Metallphosphiden in Wasserstofftransferreaktionen genannt. Die Materialien können durch unterschiedliche Verfahren hergestellt werden. Zu den Verfahren, die am weitesten verbreitet sind, gehören die Hochtemperaturreduktion von Metalloxiden (oder Phosphaten im Fall von Phosphiden) mit Reduktionsmitteln in Form von H₂, H₂/CH₄ und NH₃.

Eine Publikation von Roel Prins und Mark E. Bussell auf dem Gebiet der Metallphophide mit dem Titel "Metal Phosphides: Preparation, Characterization and Catalytic Reactivity" umfasst auch das katalytische Verhalten von Metallphophiden (siehe: CATALYSIS LETTERS, KLUWER ACADEMIC PUBLISHERS-PLENUM PUBLISHERS, Bd. 142, Nr. 12, 9. November 2012 (2012-11-09), Seiten 1413-1436, XP035145939, ISSN: 1572-879X, DOI: 10.1007/S10562-012-0929-7). Als Reaktionen werden hauptsächlich HDS, HDN und HDO untersucht. Ein Aspekt der Untersuchung betrifft auch die bimetallischen Phosphide.

US 2014/0018455 A1 offenbart phosphidhaltige Katalysatoren zur Umsetzung von Synthesegas, die ein oder mehrere Metalle aus der Gruppe Fe, Co, Ni umfassen. Das Metallphosphid bildet die Aktivkomponente, die auf einem Träger vorliegt. Als Träger dienen SiO₂ oder Al₂O₃. Bei dem katalytischen Verfahren zur Umsetzung von Synthesegas handelt es sich um ein Fischer-Tropsch-Verfahren. Beim Fischer-Tropsch-Verfahren kommt es zum Aufbau und Wachstum von Kohlenwasserstoffen, die durch Kettenwachstum gebildet werden. Die in den Beispielen gezeigten Umsetzungen werden mit einem Synthesegas durchgeführt, das ein H₂/CO-Verhältnis von 2 aufweist.

Die Patentschrift CH 314911 beschreibt ein modifiziertes Fischer-Tropsch-Verfahren, wobei ein einziges Beispiel aufgeführt wird. Gemäß dieses Beispiels wird Ethylen mit Kohlenmonoxid und Wasserdampf (CO/H₂O-Verhältnis: 2/1) an einem metallischen Kobalt-Thorium-Katalysator umgesetzt, wobei neben Diethylketon und höhersiedenden Aldehyden und Ketonen lediglich 40% Propionaldehyd im flüssigen Reaktionsprodukt erhalten werden.

Die Aufgabe ist es, ein Verfahren zur Durchführung von Hydroformylierungen in der Gasphase bereitzustellen, mittels welchem Oxoaldehyde in guter Ausbeute und Selektivität erhalten werden und bei dem die Bildung von Nebenprodukten, insbesondere die Bildung von Alkanen, möglichst gering gehalten wird.

Die Aufgabe wird dadurch gelöst, dass ein Verfahren zur Herstellung von C₃- bis C₁₉-Aldehyden und/oder Alkoholen durch die Hydroformylierung von Olefinen und/oder Alkinen in der Gasphase mit einem Gemisch aus Wasserstoff und oder Kohlenmonoxid an einem heterogenen Katalysator bei einer Temperatur von 80 - 250 °C und einem Druck von 1,5 - 50 bar über Atmosphärendruck (entsprechend dem absoluten Druck von 2,5 - 51 bar), in Anwesenheit eines Trägers und/oder von Promotoren, bereitgestellt wird, dadurch gekennzeichnet ist, dass das Gemisch aus Wasserstoff und Kohlenmonoxid ein H₂/CO-Verhältnis von 0,1 - 0,9 aufweist, der Anteil an Eduktkomponente in der Form von Olefin und/oder Alkin im Bereich von 2 - 30 % auf Volumenbasis liegt und als katalytisch aktive Komponente des heterogenen Katalysators Rh- oder Co-Phophid oder Gemische der beiden verwendet wird.

Die im Rahmen des erfindungsgemäßen Verfahrens eingesetzten Olefine und/oder Alkine stellen Substrate dar, die unter den gegeben Bedingungen des Verfahrens unter Knüpfung einer Kohlenstoff-Kohlenstoff-Bindung zur Reaktion gebracht werden. Das erfindungsgemäße Verfahren führt zur Bildung von C₃- bis C₁₉-Aldehyden und/oder Alkoholen. Somit weisen die mittels des Verfahrens hergestellten Aldehyde und/oder Alkohole drei bis neunzehn Kohlenstoffatome auf. Als Substrate werden besonders bevorzugt diejenigen Olefine eingesetzt, die aus der Gruppe Ethen, Propen, 1-Buten, 2-Buten, Isobuten, 1-Penten, 2-Methyl-1-buten, 2-Methyl-2-buten, Cyclopenten, 1-Hexen, Cyclohexen, 1-Octen, 1-Decen, 1-Dodecen, 1-Teradecen, 1-Hexadecen, Vinylaromaten oder aus der Gruppe der sogenannten Isoolefine stammen; wobei es sich bei den Olefinen aus der Gruppe der sogenannten Isoolefine um ein oder mehrere Olefine handelt, die ausgewählt sind aus der Gruppe Isoocten, Isononen, Isodecen, Isoundecen, Isododecen, Isotetradecen, Isohexadecen.

In einer bevorzugten Ausführungsform weisen die eingesetzten Substrate zumindest eine weitere funktionelle Gruppe auf, vorzugsweise ist die funktionelle Gruppe ausgewählt aus der Gruppe von Carboxyl-, Aledehyd-, Hydroxy-, Epoxy-Gruppe.

In einer bevorzugten Ausführungsform ist das Verfahren zur Herstellung von C₃- bis C₁₉-Aldehyden und/oder Alkoholen dadurch gekennzeichnet, dass das Eduktfluid zumindest ein Hilfsreagenz umfasst, wobei es sich bei dem Hilfsreagenz vorzugsweise um eine Komponente aus der Gruppe Sauerstoff, Wasser, Halogenverbindungen, Ammoniak und/oder Methanol handelt.

Beispielsweise können als Hilfsreagens eine Verbindung oder auch mehrere Verbindungen aus der Gruppe Sauerstoff, Wasser, Halogenverbindungen, Ammoniak und/oder Methanol eingesetzt werden. Neben Methanol können auch andere organische Verbindungen eingesetzt werden. Die Hilfsreagenzien haben einen unterstützenden Einfluss auf den Ablauf des Verfahrens zur Herstellung von C₃- bis C₁₉-Aldehyden und/oder Alkoholen, wobei diese jedoch nicht direkt an der Knüpfung der Kohlenstoff-Kohlenstoff-Bindungen teilnehmen.

Halogenverbindungen, die als Hilfsreagenzien eingesetzt werden, können unter anderem sein: Halogenwasserstoffe, organische Halogenverbindungen und andere dem Fachmann bekannte Verbindungen der Halogene sowie auch Halogene in elementarer Form. Der Begriff Halogene in elementarer Form bedeutet, dass darin Moleküle aus der Gruppe F₂, Cl₂, Br₂ und/oder J₂ umfasst sind.

Die Durchführung des Verfahrens in Gegenwart von Wasser als Hilfsreagenz kann mehrere nützliche Funktionen haben, die nachfolgend näher ausgeführt werden. Die Ausführung stellt keine Einschränkung der Erfindung dar. Durch die Anwesenheit von Wasser kann die Desorption von Produkten und/oder Nebenprodukten erleichtert werden. Durch das Wasser können die Metallspezies beim Wechsel des Valenzzustands innerhalb der katalytisch aktiven Komponente unterstützt werden. Durch die Zugabe von Wasser kann die Bildung von aktiven Zentren innerhalb der Phosphidverbindung gesteuert, wobei die Bildung von aktiven Zentren unterstützt oder unterdrückt werden kann.

Der Begriff Kohlenmonoxid-Präkursor bezieht sich drauf, dass das Kohlenmonoxid zunächst nicht in freier Form im Eduktfluid vorliegt, sondern dem Eduktfluid als Vorläuferverbindung zugeführt wird, die erst im Reaktionsraum freiem Kohlenmonoxid umgewandelt wird. Somit kann das Kohlenmonoxid dem Eduktfluid auch in der Form eines Kohlenmonoxid-Präkursors zugeführt werden. In einer bevorzugten Ausführungsform des Verfahrens wird das Kohlenmonoxid dem Eduktstrom als freies Kohlenmonoxid zugeführt.

In einer bevorzugten Ausführungsform umfasst das erfindungsgemäße Verfahren neben der Verwendung von Hilfsreagenzien - wie beispielsweise Sauerstoff, Wasser, Ammoniak und/oder organische Verbindungen wie beispielsweise Methanol. Die Hilfsreagenzien können zusammen mit Kohlenmonoxid und dem zumindest einen Substrat reagieren, müssen aber nicht selber an der Reaktion teilnehmen. Bei den Substraten handelt es sich um zumindest ein Olefin und/oder ein Alkin. Beispiele für solche Reaktionen sind unter anderem die Hydroformylierung von Olefinen mit Kohlenmonoxid und Wasserstoff zu Aldehyden, oder die Hydroxycarbonylierung von Olefinen zu Estern mit Methanol und Kohlenmonoxid. Weitere Beispiele für den Einsatz von Hilfsreagenzien sind die Umsetzung von Kohlenmonoxid und Methanol in der Gegenwart von Sauerstoff zu Dimethylcarbonat. Der Sauerstoff bewirkt eine Re-oxidation des Katalysators beziehungsweise der der katalytisch aktiven Komponente des Katalysators. Das Hilfsreagenz Wasser kann nützlich sein, da es die Desorption von Nebenprodukten und/oder Produkt erleichtern kann. Weiterhin kann das Wasser dazu beitragen, dass einzelne Metalle des Phosphid-Katalysators den Valenzzustand wechseln. Darüber hinaus können bestimmte aktive Zentren durch die Anwesenheit von Wasser unterdrückt oder erst gebildet werden.

Beispiele für Produkte, die im Rahmen des erfindungsgemäßen Verfahrens über Fischer-Tropsch-ähnliche Mechanismen erhalten werden können, sind unter anderem Paraffine, isoParaffine, Olefine, insbesondere endständige Olefine, sowie iso-Olefine und verzweigte Olefine, höhere Alkohole mit mehr als einem Kohlenstoffatom. Solche Reaktionen können, wie aus der Literatur für andere Katalysatorsysteme bekannt ist, auch Verbindungen als Produkte haben wie Säuren, Ester, Aldehyde, Ketone und/oder Aromaten.

Beispiele für Ausführungsformen des erfindungsgemäßen Verfahrens sind unter anderem die Herstellung von internen und endständigen ungesättigten Aldehyden durch Hydroformylierung von Acetylenderivaten, die Herstellung von zu internen und endständigen Aldehyden durch Hydroformylierung von Olefinen.

Weitere Beispiele sind die Herstellung von Essigsäure durch Carbonylierung von Methanol, die Herstellung von Methylacetat durch Carbonylierung von Dimethyether, die Herstellung von Acetanhydrid durch Carbonylierung von Methylacetat, die Herstellung von Dimethylglyoxal durch die Carbonylierung von Nitromethan, die Herstellung von Glykolaldehyd und/oder Ethylenglykol durch die Carbonylierung von Formaldeyd und/oder einem Acetal des Formaldehyds, und/oder von Trioxan, die Herstellung von aromatischen Aldehyden und Säurederivaten durch Koch-Carbonylierung von Aromaten, Herstellung von Isocyananten und/oder Carbamaten durch die Carbonylierung von Aminen, Herstellung von Isocyananten und/oder Carbamaten durch die Carbonylierung von Nitro- oder Nitrosoverbindungen, die Herstellung von Säuren und Estern durch die Carboxymethylierung von Olefinen, die Herstellung von Lactonen wie Propiolacton durch die Carbonylierung von Epoxiden wie Ethylenoxid.

Im Rahmen der Erfindung sind insbesondere diejenigen Ausführungsformen des Verfahrens zur Hydroformylierung von Kohlenwasserstoffen eingeschlossen, die die Herstellung von Aldehyden oder Säurederivaten aus Olefinen betreffen.

Im Rahmen der Erfindung sind insbesondere diejenigen Ausführungsformen des Verfahrens zur Herstellung von C₃- bis C₁₉-Aldehyden und/oder Alkoholen eingeschlossen, bei denen ein Olefin als Substrat mit einem Gemisch aus Wasserstoff und reagiert und dabei eine Kohlenstoff-Kohlenstoff-Bindung mit der olefinischen Bindung gebildet wird.

Vorzugsweise weisen die zur Hydroformylierung eingesetzten Olefine2 bis 8 C-Atome auf, weiter vorzugsweise 2 bis 4 C-Atome und besonders bevorzugt 2 - 3 C-Atome. Die für das Verfahren eingesetzten Olefine können lineare, cyclische oder verzweigte Struktur oder Strukturelemente aufweisen. Die cyclischen Strukturelemente können aromatischen und/oder nichtaromatischen Charakter haben. Die Olefine können ebenfalls andere funktionelle Gruppen enthalten - wie beispielsweise organische Säuregruppen, organische Ketofunktionen oder anorganische Estergruppen oder andere dem Fachmann bekannte Funktionalitäten.

Besonders bevorzugt sind Ausführungsformen des erfindungsgemäßen Verfahrens, bei denen die Umsetzung von Ethylen zu Propionaldehyd erfolgt beziehungsweise bei denen die Umsetzung von Propylen zu n- und/oder iso-Butyraldehyd erfolgt.

Ebenso mit eingeschlossen im Rahmen des erfindungsgemäßen Verfahrens ist es, dass der Herstellung der Aldehyde ein weiterer Verfahrensschritt nachgeschaltet ist. Vorzugsweise erfolgt eine Hydrierung als nachgeschaltetem Verfahrensschritt. In dieser besonders bevorzugten Ausführung des Verfahrens führt die Hydroformylierung von Ethylen zur Bildung von Propanol beziehungsweise die Hydroformylierung von Propylen führt zur Bildung von n- und/oder isoButanol.

Auch eingeschlossen im Rahmen der Erfindung ist die Ausführungsform bei der die Substrate bzw. die aus den Substraten gebildeten Aldehyde im Anschluss an die Hydroformylierung teilweise oder vollständig zu den korrespondierenden Alkoholen hydriert werden. Diese Hydrierung kann entweder ebenso wie die Hydroformylierung mittels der erfindungsgemäßen heterogenen Katalysatoren erfolgen, oder mittels anderer Katalysatormaterialien, die dem erfindungsgemäßen Katalysator nachgeschaltet sind. Dabei können sich die nachgeschalteten Katalysatoren auch im gleichen Reaktionsraum befinden wie der erfindungsgemäße Katalysator.

Der Begriff Bestandteile des Eduktfluids bezieht sich auf die eingesetzten Substrate, die Hilfsreagenzien und Kohlenmonoxid. Davon abgesehen kann das Eduktfluid auch Trägerfluid aufweisen, das nicht an der Reaktion beteiligt ist.

Unter dem Begriff Produkt beziehungsweise Produktfluid sind die Reaktionsprodukte sowie auch Teile des Eduktfluids zu verstehen. Da die eingesetzten Substrate zum Teil nicht vollständig umgesetzt werden können.

Bei dem erfindungsgemäßen Verfahren zur Herstellung von C₃- bis C₁₉-Aldehyden und/oder Alkoholen sind auch diejenigen Verfahrensvarianten eingeschlossen, bei denen das Kohlenmonoxid erst während des Verfahrens aus der Eduktkomponente gebildet wird.

Weiterhin ist in Bezug auf die Durchführung des erfindungsgemäßen Verfahrens zur Herstellung von C₃- bis C₁₉-Aldehyden und/oder Alkoholen festzustellen, dass es hierbei bevorzugt ist, dass der heterogene Katalysator als heterogener Festbettkatalysator eingesetzt wird. Vorzugsweise wird der heterorgene Festbettkatalysator in einer gas/fest oder gas/flüssig/fest Reaktion eingesetzt. Gasphase bedeutet, dass die Edukte in gasförmiger Form vorliegen. In Abhängigkeit der jeweiligen Reaktionsbedingungen ist es nicht ausgeschlossen, dass zumindest ein Teil der Olefine und/oder der Produkte an der Oberfläche oder in den Poren des Katalysators auskondensiert. Somit ist es nicht ausgeschlossen, dass an dem erfindungsgemäßen Verfahren und in Verbindung mit der Reaktion an dem Festbettkatalysator auch ein Teil an flüssigen Phasen beteiligt ist, die in Verbindung mit der Auskondensation und der Filmbildung auf der Katalysatoroberfläche oder in den Katalysatorporen auftreten.

In einer bevorzugten Ausführungsform kann das Verfahren so geführt werden, dass sowohl die Edukte als auch die Produkte in gasförmiger Form vorliegen. Erfindungsgemäß werden hier Druck und Temperatur so gewählt, dass die Taupunktslinien unter den Betriebsbedingungen nicht überschritten werden. In einer besonderen Ausführungsform kommt es hierbei zu einer teilweisen Auskondensation von Edukten und Produkten.

In einer weiteren Ausführungsform kann das Verfahren so geführt werden, dass die Edukte gasförmig und die Produkte in flüssiger Form vorliegen. In dieser Ausführungsform werden Druck und Temperatur so gewählt, dass die Taupunktslinien für die Edukte unter den Betriebsbedingungen nicht überschritten werden, eine komplette oder teilweise Auskondensation der Produkte wird erlaubt.

In einer weiteren Ausführungsform, die auch bevorzugt ist, kann das Verfahren so geführt werden, dass die Produkte gasförmig und die Edukte in flüssiger Form vorliegen. In dieser Ausführungsform werden hier Druck und Temperatur so gewählt, dass die Taupunktslinien für die Produkte unter den Betriebsbedingungen nicht überschritten werden, eine komplette oder teilweise Auskondensation der Edukte wird erlaubt.

Die Ausführungsform des Verfahrens kann so gestaltet werden, dass sowohl die Edukte als auch die Produkte in flüssiger Form vorliegen. In dieser Ausführungsform werden Druck und Temperatur so gewählt, dass die Taupunktslinien unter den Betriebsbedingungen überschritten werden. In einer besonderen Ausführungsform kommt es hierbei nur zu einer teilweisen Auskondensation von Edukten und Produkten.

In einer bevorzugten Ausführungsform wird das Verfahren als Festbettverfahren in der Fahrweise fester Katalysator - gasförmige Produkte und Edukte durchgeführt.
In einer weiteren Ausführungsform kann das Verfahren als Festbettverfahren in der Fahrweise fester Katalysator - flüssige Produkte und gasförmige Edukte durchgeführt werden. Eine solche Fahrweise wird auch als Gas-to-Liquid-Verfahren bezeichnet. Diese Ausführungsform kann auch als Rieselbett betrieben werden, bei dem die Reaktorgestaltung und Betriebsweise die Ausbildung eines Rieselbettes gestattet.

In einer weiteren Ausführungsform kann das Verfahren auch als Festbettverfahren in der Fahrweise fester Katalysator - gasförmige Produkte und flüssige Edukte durchgeführt werden. Eine derartige Ausführungsform des Verfahrens wird als Liquid-to-Gas-Verfahren bezeichnet. Diese Ausführungsform kann auch als Rieselbett betrieben werden, wobei die Reaktorgestaltung und Fahrweise die Ausbildung eines Rieselbettes gestattet.

In einer bevorzugten Ausführungsform wird das Verfahren als Festbettverfahren in der Fahrweise fester Katalysator - flüssige Produkte und flüssige Edukte durchgeführt. In dieser Ausführungsform wird das erfindungsgemäße Verfahren somit in flüssiger Phase durchgeführt. Eine Zufuhr der Eduktfluide in flüssiger Form kann dabei beispielsweise auch über eine Sättigung eines geeigneten Lösungsmittels mit dem Eduktgas geschehen. Eine derartige Ausführung wird auch als Sumpffahrweise bezeichnet. In einer besonderen Ausführungsform kann das Verfahren auch als Rieselbett betrieben werden, in welchem die Reaktorgestaltung und Fahrweise die Ausbildung eines Rieselbettes gestattet.

Weiterhin kann das Verfahren als Wirbelschichtverfahren oder als Simulated-Moving-Bed-Verfahren betrieben werden, wobei hier der heterogene Katalysator mit der katalytisch aktiven Komponente in dieser Fahrweise als fester Katalysator und die Produkte und Edukte vorzugsweise gasförmig vorliegen.

Das Verfahren zur Herstellung von C₃- bis C₁₉-Aldehyden und/oder Alkoholen dadurch gekennzeichnet ist, dass der Druck beim Inkontaktbringen von Eduktfluid und heterogenem Katalysator im Bereich von 1,5 - 50 bar liegt über dem Atmosphärendruck (entsprechend 2,5 - 51 bar absoluter Druck), vorzugsweise von 5 - 50 bar über dem Atmophärendruck. Die hier genannten Druckangaben beziehen sich auf den Druck oberhalb des Atmosphärendrucks.

Das Inkontaktbringen des Eduktfluids und des Katalysators mit der katalytisch aktiven Komponente erfolgt bei einer Temperatur, die im Bereich von 80 - 250 °C liegt, vorzugsweise von 100 - 230 °C.

Besonders bevorzugt ist bei dieser Ausführungsform des erfindungsgemäßen Verfahrens die Durchführung als Festbettverfahren in der Fahrweise fester Katalysator - gasförmige Produkte und Edukte. Eine entsprechende Abstimmung des Temperatur- und Druckbereiches zur Vermeidung des Traupunktes der Edukte und Produkte ist ebenfalls erfindungsgemäß.

Das erfindungsgemäße Verfahren zur Herstellung von C₃- bis C₁₉-Aldehyden und/oder Alkoholen durch Hydroformylierung von Olefinen und/oder Alkinen in der Gasphase wird bei einem Druck im Bereich von 1,5 - 50 bar über dem Atmosphärendruck , weiter vorzugsweise von 5 - 50 bar über dem Atmosphärendruck, wobei die Temperatur Reaktion im Bereich 80 - 250 °C liegt, vorzugsweise von 100 - 230 °C. Die Druckangaben beziehen sich auf Drücke, die oberhalb des Atmosphärendrucks liegen.

In einer bevorzugten Ausführungsform wird das Verfahren zur Herstellung von C₃- bis C₁₉-Aldehyden und/oder Alkoholen in der Gegenwart des erfindungsgemäßen Katalysators bei einer Temperatur von 100 - 230 °C.

Das erfindungsgemäße Verfahren zur Herstellung von C₃- bis C₁₉-Aldehyden und/oder Alkoholen in Gegenwart von einem Gemisch aus Wasserstoff und Kohlenmonoxid wird bei einem H₂/CO-Verhältnis im Bereich von 0,1 - 0,9 durchgeführt. Vorzugsweise ist das H₂/CO-Verhältnis im Bereich 0,5 - 0,9 und ganz besonders bevorzugt ist das H₂/CO-Verhältnis im Bereich von 0,7 - 0,9.

In einer bevorzugten Ausführungsform betrifft die Erfindung ein Verfahren zur Herstellung von C₃- bis C₅ -Aldehyden und/oder Alkoholen durch die Hydroformylierung von C₂- bis C₄- Olefinen und/oder Alkinen mit einem Gemisch aus Wasserstoff und Kohlenmonoxid. Darüber hinaus bevorzugt betrifft die Erfindung ein Verfahren zur Herstellung von C₃- bis C₄-Aldehyden und/oder Alkoholen durch die Hydroformylierung von C₂- bis C₃- Olefinen und/oder Alkinen mit einem Gemisch aus Wasserstoff und Kohlenmonoxid.

Vorzugsweise wird das Verfahren zur Herstellung von C₃- bis C₁₉-Aldehyden und/oder Alkoholen in der Gegenwart des erfindungsgemäßen Katalysators bei einem Anteil an Hilfsreagenz von 2 - 40 % auf Volumenbasis durchgeführt, ganz besonders bei einem Anteil an Hilfsreagenz von 3 - 20 % auf Volumenbasis durchgeführt.

In einer weiteren Ausführungsform, die ebenso bevorzugt ist, wird das Verfahren unter Verwendung von Wasser als Hilfsreagenz ausgeführt.

### I. Heterogener Katalysator mit katalytisch aktiver Komponente

Ein Aspekt der erfindungsgemäßen Lösung betrifft die Bereitstellung eines heterogenen Katalysators mit einer katalytisch aktiven Komponente zur Herstellung von C₃- bis C₁₉-Aldehyden und/oder Alkoholen durch die Umsetzung von Olefinen und/oder Alkinen, wobei der Katalysator dadurch gekennzeichnet ist, dass als katalytisch aktive Komponente Rh- oder Co-Phosphid oder Gemische der beiden verwendet wird und der heterogen Katalysator in Anwesenheit eines Trägers und/oder von Promotoren vorliegt.

Es ist festzustellen, dass für die Durchführung des Verfahrens zur Herstellung von den C₃- bis C₁₉-Aldehyden und/oder Alkoholen vorzugsweise und erfindungsgemäß diejenigen Katalysatoren in den Ausführungsformen eingesetzt werden, wie sie in der vorliegenden Beschreibung näher ausgeführt werden. Sofern besonders bevorzugte Eigenschaften der Katalysatoren genannt werden, so kann auch die Verwendung dieser Katalysatoren bei der Durchführung des erfindungsgemäßen Verfahrens besonders bevorzugt sein. Ein Aspekt des Verfahrens betrifft somit das Verfahren in Gegenwart der katalytisch aktiven Komponenten, wobei als katalytisch aktive Komponente ein spezielles Phosphid verwendet. In weiteren Ausführungsform ist es bevorzugt, das Verfahren mit einem Katalysator durchgeführt, bei dem die katalytisch aktive Komponente in Gegenwart eines bevorzugten Trägermaterials vorliegt.

Somit ist es bevorzugt, dass die katalytisch aktive Komponente in Gegenwart eines Trägers vorliegt, weiter bevorzugt liegt die katalytisch aktive Komponente in Gegenwart eines kohlenstoffhaltigen Trägermaterials vor. Weiter vorzugsweise umfasst der Träger zumindest ein Material aus der Gruppe Carbide, kohlenstoffhaltige Träger. Weiter vorzugsweise umfasst der Katalysator einen Träger. Vorzugsweise umfasst der Träger ein Material aus der Gruppe Aktivkohle, Graphit und/oder Siliziumcarbid. In Bezug auf das erfindungsgemäße Verfahren wurde gefunden, dass die Kombination der bevorzugten Trägermaterialien mit bevorzugtem als katalytisch aktiver Komponente des heterogenen Katalysators zu einer weiteren Leistungssteigung führt gegenüber einem Katalysator, der nicht das bevorzugte Trägermaterial umfasst.

Ein Aspekt der Erfindung ist somit auch ein Katalysator mit einer katalytisch aktiven Komponente, bei dem als katalytisch aktive Komponente Rh- oder Co-Phosphid oder Gemische der beiden, vorzugsweise Rh-Phosphid, verwendet wird.

Im Fall, dass der Phosphor mit genau einem Element aus der hier aufgeführten Gruppe verbunden ist, so handelt es sich um eine binäre Verbindung. Im Fall, dass der Phosphor mit zwei Elementen Verbunden ist handelt es sich um eine ternäre Verbindung. Darüber hinaus kann es sich bei dem Phosphid jedoch auch um eine quarternäre beziehungsweise eine polynäre Verbindung handeln. Die einzelnen Verbindungen (d.h. die unterschiedlichen binären Verbindungen beziehungsweise auch die polynären Verbindungen) können dabei als gemischte Phosphide in einer gemeinsamen kristallinen oder röntgenamorphen Struktur vorliegen.

Es ist nicht ausgeschlossen, dass die katalytisch aktive Komponente in Gegenwart von Promotoren vorliegt. Promotoren sind solche Zusatzstoffe, die zu einer Steigerung der Selektivität und/oder der Aktivität des Katalysators führen. Somit sind auch diejenigen Phosphide in die Gruppe der Promotoren eingeschlossen, die nicht die Phosphide der katalytisch aktiven Komponente bilden. Hierbei mit eingeschlossen sind auch Mischphosphide. Beispielsweise liegen Mischphosphide, die Platinphosphid enthalten, in der Form von Pt₅HgP vor und Mischphosphide, die Iridiumphosphid enthalten, in der Form von IrTeP vor.

Der erfindungsgemäße Katalysator liegt in An- oder Abwesenheit eines Trägermaterials vor. Vorzugsweise liegt der heterogene Katalysator als Trägerkatalysator vor, bei dem die katalytisch aktive Komponente des Katalysators mit einem Träger verbunden ist oder auf einem Träger abgeschieden vorliegt.

Die Erfindung betrifft einen heterogenen Katalysator, der dadurch gekennzeichnet ist, dass als katalytisch aktive Komponente Rh- oder Co-Phosphid oder Gemische der beiden, vorzugsweise Rh-Phosphid, verwendet wird. Der heterogene Katalysator kann in der Gegenwart von Promotoren vorliegen. Als Promotoren können auch andere Phosphide geeignet sein, bei denen dass Phosphid in Gegenwart von einem oder mehreren Elementen aus der Gruppe Sc, Y, La, der Lanthaniden, Ti, Zr, Hf, V, Nb, Ta, Cr, Mo, W, Mn, Re, Fe, Ru, Os, Ni, Cu, Ag, Au, Zn, Cd, Hg B, Al, Ga, In, Ge, Si, Sn, Pb, Sb, As, Se, Te vorliegt.

Vorzugsweise weist der Katalysator Nanopartikel auf, die die katalytisch aktive Komponente enthalten. Die Begriffe Nanopartikel, nanopartikulär beziehungsweise feinteilig bezieht sich auf Partikel, deren Größe in einem Bereich von 1 - 100 nm liegt, vorzugsweise liegt die Größe der Partikel in einem Bereich von 2 - 60 nm, darüber hinaus bevorzugt liegt die Größe der Partikel in einem Bereich von 3 - 30 nm. Darüber hinaus ist es bevorzugt, dass die Partikel in hochdisperser Form über den Katalysatorträger verteilt vorliegen.

Somit ist der Katalysator vorzugsweise dadurch gekennzeichnet, dass der Katalysator Partikel aufweist, die die katalytisch aktive Komponente enthalten, wobei die Größe der Partikel im Bereich von 1 - 100 nm liegt, vorzugsweise im Bereich von 2 - 60 nm, weiter vorzugsweise im Bereich 3-30 nm.

Die Partikel können eine eher einheitliche Größe oder eher unterschiedliche Größen aufweisen. Beispielsweise kann die Größenverteilung der Partikel mittels TEM-Aufnahmen in Kombination mit Bildauswertungsverfahren ermittelt werden. Möglich ist, dass die Partikel eine monomodale Größenverteilung oder eine mehrmodale Größenverteilung aufweisen. Bevorzugt ist es, dass die feinen Teilchen beziehungsweise die Partikel mit der katalytisch aktiven Komponente in hochdisperser Form vorliegen.

In einer bevorzugten Ausführungsform ist der Katalysator mit einer katalytisch aktiven Komponente zur Herstellung von C₃- bis C₁₉-Aldehyden und/oder Alkoholen dadurch gekennzeichnet, dass die Partikel einen Anteil an katalytisch aktiver Komponente aufweisen, der im Bereich von 10 - 100 Gew.-% liegt, weiter vorzugsweise liegt der Anteil an katalytisch aktiver Komponente in den Partikeln im Bereich von 20 - 95 Gew.-%, darüber hinaus bevorzugt liegt der Anteil an katalytisch aktiver Komponente in den Partikeln im Bereich von 30 - 90 Gew.-%.

Die Phosphide können entweder in kristalliner Form, teilkristalliner Form oder in röntgenamorpher Form vorliegen. Davon abgesehen können ein Teil der Phosphide in kristalliner Form, teilkristalliner und ein Teil in röntgenamorpher Form vorliegen. Bevorzugt ist es, dass die Phosphide, die als katalytisch aktive Komponente verwendet werden, in kristalliner Form vorliegen. Die Struktur lässt sich mittels Röntgendiffraktometrie oder Elektronenbeugung im Transmissionselektronenmikroskop (TEM) bestimmen.

Ausführungsformen von Phosphidverbindungen, die möglicherweise Eignung als Promotoren aufweisen können. Bevorzugt sind die Phosphide der Elemente aus der Gruppe Sc, Y, La, der Lanthaniden, Ti, Zr, Hf, V, Nb, Ta, Cr, Mo, W, Mn, Re, Fe, Ru, Os, Ni, Cu, Ag, Au, Zn, Cd, B, Al, Ga, In, Ge, Si, Sn, As, besonders bevorzugt sind die Phosphide der Elemente der Gruppe Cr, Mo, W, Mn, Re, Fe, Ru, Os, Ni, Cu, Ag, Au, B, Al, Ga, In, Ge, Sn, ganz besonders bevorzugt sind die Phosphide der Elemente der Gruppe Mn, Re, Fe, Ru, Os, Co, Ni, Cu, Ag, Au, B.

Mögliche Eignung als Promotoren besitzen die kristallinen oder nano-kristallinen Phosphide der Gruppe: SC₃P, ScP, SC₃P₂, SC₇P₃, Ti₃P, Ti₂P, Ti₄P₃, Ti₅P₃, Ti₇P₄, TiP, V₃P, V₂P, V₁₂P₇, V₅P₃, V₄P₃ VP, VP₂, Cr₃P, Cr₁₂P₇, Cr₂P, CrP, CrP₂, CrP₄, Mn₃P, Mn₂P, MnP, MnP₄, Fe₄P, Fe₃P, Fe₂P, FeP, FeP₂, FeP₄, Ni₃P, Ni₈P₃, Ni₅P₂, Ni₁₂P₅, Ni₂P, Ni₅P₄, NiP, NiP₂, NiP₃, Cu₃P, CuP₂, Cu₂P₇, Zn₃P₂, ZnP₂, ZnP₄, B₆P, B₁₃P₂, BP, AlP, GaP, SiP, SiP₂, GeP, GeP₃, YP, YP₅, Zr₃P, Zr₂P, Zr₇P₄, Zr₁₄P₉, ZrP ZrP₂, Nb₃P, Nb₇P₄, Nb₅P₃, Nb₈P₅, NbP, NbP₂, Nb₄P₇, Nb₂P₅, Mo₃P, Mo₄P₃, Mo₈P₅, MoP, MoP₂, Ru₂P, RuP, RuP₂, RuP₃, RuP₄, AgP₂, Ag₃P₁₁, Au₂P₃, Cd₃P₂, Cd₇P₁₀, CdP₂, CdP₄, InP, InP₃, Sn₄P₃, SnP, Sn₃P₄, SnP₃, nP (Ln = La, Ce, Pr, Nd, Sm, Gd, Tb, Dy, Ho, Er, Tm, Yb, Lu), Ln₃P₄ (Ln = Eu) LnP₂ (Ln = La, Ce, Pr), LnP₃ (Ln = Eu) LnP₅ (Ln = La, Ce, Pr, Nd, Sm, Gd, Dy, Ho, Tm, Yb, Lu), LnP₇ (Ln = La, Eu) Hf₃P, Hf₂P, Hf₃P₂, Hf₂₇P₁₆, Hf₇P₄, HfP, HfP₂, Ta₃P, Ta₂P Ta₅P₃, TaP, TaP₂, W₃P, WP, WP₂, Re₂P Re₃P₄, Re₆P₁₂, Re₂P₅, ReP₃, ReP₄, OsP₂, OsP₄,.

Als katalytisch aktive Komponente sind die kristallinen oder nanokristallinen Phosphide aus der Gruppe Co₂P, CoP, CoP₃, Rh₂P, Rh₁₂P₇, Rh₃P₂, Rh₄P₃, RhP₂, RhP₃ bevorzugt.

Mögliche Eignung als Promotoren besitzen die kristallinen oder nano-kristallinen Phosphide der Gruppe: Ti₃P, Ti₂P, Ti₄P₃, Ti₅P₃, Ti₇P₄, TiP, V₃P, V₂P, V₁₂P₇, V₅P₃, V₄P₃, VP, VP₂, Cr₃P, Cr₁₂P₇, Cr₂P, CrP, CrP₂, CrP₄, Mn₃P, Mn₂P, MnP, MnP₄, Fe₄P, Fe₃P, Fe₂P, FeP, FeP₂, FeP₄, Ni₃P, Ni₈P₃, Ni₅P₂, Ni₁₂P₅, Ni₂P, Ni₅P₄, NiP, NiP₂, NiP₃, CU₃P, CuP₂, Cu₂P₇, Zn₃P₂, ZnP₂, ZnP₄, B₆P, B₁₃P₂, BP, AlP, GaP, GeP, GeP₃, Mo₃P, Mo₄P₃, Mo₈P₅, MoP, MoP₂, Ru₂P, RuP, RuP₂, RuP₃, RuP₄, AgP₂, Ag₃P₁₁, Au₂P₃, Cd₃P₂, Cd₇P₁₀, CdP₂, CdP₄, InP, InP₃, Sn₄P₃, SnP, Sn₃P₄, SnP3, W₃P, WP, WP₂, Re₂P Re₃P₄, Re₆P₁₂, Re₂P₅, ReP₃, ReP₄, OsP₂, OsP₄,. Mögliche Eignung als Promotoren besitzen weiterhin die kristallinen oder nano-kristallinen Phosphide der Gruppe: Ti₃P, Ti₂P, Ti₄P₃, Ti₅P₃, Ti₇P₄, TiP, V₃P, V₂P, V₁₂P₇, V₅P₃, V₄P₃ VP, VP₂, Cr₃P, Cr₁₂P₇, Cr₂P, CrP, CrP₂, CrP₄, Mn₃P, Mn₂P, MnP, MnP₄, Fe₄P, Fe₃P, Fe₂P, FeP, FeP₂, FeP₄, Ni₃P, Ni₈P₃, Ni₅P₂, Ni₁₂P₅, Ni₂P, Ni₅P₄, NiP, NiP₂, NiP₃, Cu₃P, CuP₂, Cu₂P₇, B₆P, B₁₃P₂, BP, Mo₃P, Mo₄P₃, Mo₈P₅, MoP, MoP₂, Ru₂P, RuP, RuP₂, RuP3, RuP4, AgP₂, Ag₃P₁₁, Au₂P₃, Cd₃P₂, Cd₇P₁₀, CdP₂, CdP₄, InP, InP₃, Sn₄P₃, SnP, Sn₃P₄, SnP₃, W₃P, WP, WP₂, Re₂P Re₃P₄, Re₆P₁₂, Re₂P₅, ReP₃, ReP₄, OsP₂, OsP₄,.

Die Promotoren können auch Metalle oder Nichtmetalle umfassen. Beispielsweise ist es denkbar, dass als Promotoren ein oder mehrere Metalle aus der Gruppe Sc, Y, La, der Lanthaniden, Ti, Zr, Hf, V, Nb, Ta, Cr, Mo, W, Mn, Re, Fe, Ru, Os, Ni, Cu, Ag, Au, Zn, Cd, Hg B, Al, Ga, In, Ge, Si, Sn, Pb, Sb, As, Se verwendet werden. Als Nichtmetalle kann beispielsweise Bor enthalten sein.

Es können Teile der Promotorenbeispielsweise in elementarer Form, als Oxide, als Hydroxide, als Hydride, als salzartige Verbindungen oder als andere Verbindungen vorliegen.

Das für das erfindungsgemäße Verfahren eingesetzte Katalysatormaterial kann auch mit bestimmten Elementen dotiert werden, dabei können ein oder mehrere Elemente zum Einsatz kommen. Die Dotierelemente können ebenfalls als Phosphid gleicher oder anderer Struktur vorliegen, oder in die Struktur des hauptsächlich vorliegenden Phosphides eingebaut sein, oder Teil eines polynären Phosphids sein. Beispiele für solche polynären Phosphide sind, BaRh₂P₂, CaRh₂P₂, Ca₄Rh₁₉P₁₂, Ca₆Rh₃₀P₁₉, MgRh₆P₄, SeRhP, Si₃RhP₃, BaIr₂P₂, BalrP₁, Ca₂Ir₁₂P₇, IrTeP, lnPt₅P, Pt₅HgP, IrTeP und weitere der Literatur bekannte polynäre Phosphide. Das oder die Dotierelemente können aber auch in anderer Form auf dem Katalysator vorliegen. Bevorzugt ist die Gruppe der Dotierelemente ausgewählt aus der Gruppe der Alkalimetalle, Erdalkalimetalle, Si, Ge, Hg Pb, Sb, As, S, Se, Te, F, Cl, Br, J.

In einer bevorzugten Ausführungsform ist der Katalysator mit der katalytisch aktiven Komponente für das Verfahren zur Herstellung von C₃- bis C₁₉-Aldehyden und/oder Alkoholen durch die Hydroformylierung von Olefinen und/Alkinen in der Gasphase dadurch gekennzeichnet, dass der Katalysator eine Dotierung aufweist, wobei die Dotierung Verbindungen und/oder Elemente aus der Gruppe der Alkalimetalle, Erdalkalimetalle, Si, Ge, Pb, Sb, As, S, Se, Te, F, Cl, Br, J umfasst, vorzugsweise weist die Aktivkomponente ein Verhältnis an Stoffmenge an Dotierungselementen (n_{Dot}) zu Stoffmenge an Phosphor, die im Katalysator enthalten ist (np) auf, das im Bereich von 1/10⁵ -1/1 liegt, bevorzugt liegt das Verhältnis von n_{Dot} zu np im Bereich von 1/10⁴ - 1/10, ganz besonders bevorzugt liegt das Verhältnis von n_{Dot} zu np im Bereich von 1/10³ - 1/20. Mittels der Dotierung können die katalytischen Eigenschaften des erfindungsgemäßen Verfahrens zur Herstellung der Aldehyde und/oder Alkohole zum Teil weiter gesteigert werden gegenüber denjenigen Katalysatoren, die keine Dotierung aufweisen.

In einer bevorzugten Ausführungsform ist der Katalysator mit der katalytisch aktiven Komponente für das Verfahren zur Herstellung von C₃- bis C₁₉-Aldehyden und/oder Alkoholen dadurch gekennzeichnet ist, dass die Partikel einen Anteil an katalytisch aktive Komponente aufweisen, der im Bereich von 10 - 100 Gew.-% liegt, weiter vorzugsweise liegt der Anteil an katalytisch aktiver Komponente in den Partikeln im Bereich von 20 - 95 Gew.-%, darüber hinaus bevorzugt liegt der Anteil an katalytisch aktiver Komponente in den Partikeln im Bereich von 30 - 90 Gew.-%.

Das Katalysatormaterial kann als Voll- oder Trägerkatalysator vorliegen. Wobei das Vorliegen als Trägerkatalysator bevorzugt ist. Der Begriff Trägerkatalysator umfasst dabei ein Träger und eines oder mehrere Phosphide, die als Kompositmaterial den Trägerkatalysator bilden. Es ist nicht ausgeschlossen, dass der Katalysator in Form von einer Abmischung vorliegt, die sowohl einen Vollkatalysator als auch einen Trägerkatalysator umfasst.

Besonders bevorzugt ist der erfindungsgemäße Katalysator mit einer katalytisch aktiven Komponente zur Herstellung von C₃- bis C₁₉-Aldehyden und/oder Alkoholen in einer Ausführungsform, bei der Katalysator als Trägerkatalysator vorliegt. Darüber hinaus ist es bevorzugt, dass der Träger eine kohlenstoffhaltige Verbindung enthält. Insbesondere bevorzugt ist es, dass der Träger eine Aktivkohle, Graphit und/oder Siliziumcarbid umfasst.

Die Träger können kristallin und/oder röntgenamorph vorliegen. Als Träger sind unter anderen die folgenden Materialien im Rahmen der Erfindung miteingeschlossen:
kohlenstoffhaltige Träger wie Aktivkohlen, Graphite, Kohlenstoffgerüstverbindungen wie Kohlenstoffnanoröhren (Carbon Nanotubes), Fullerene und andere bekannte Kohlenstoffgerüstverbindungen. Graphen und graphenhaltige Materialien, mit anderen Elementen modifizierte kohlenstoffhaltige Träger, Einlagerungsverbindungen des Graphit, Carbide wie Borcarbid, SiliziumCarbid, Wolframcarbid, oder andere bekannte Metallcarbide. Ebenfalls mit eingeschlossen sind Nitride wie Bornitrid, Siliziumnitrid, oder andere dem Fachmann bekannte Nitride, Boride, Silizide, Phosphide, Antimonide, Arsenide, Sulfide, Selenide, Telluride, sowie salzartige Halogenide der Haupt und Nebengruppen Elemente. Auch elementare Metalle, Halbmetalle und Nichtmetalle, geordnete, ungeordnete und eutektische Legierungen, sowie binäre und polynäre intermetallische Phasen sind als Träger miteingeschlossen.

Ebenfalls mit eingeschlossen sind sulfidische Träger. Beispiele sind unter anderem die Sulfide des Eisens, des Molybdäns oder des Wolframs.

Ebenfalls mit eingeschlossen sind organische Polymere wie Polyolefine, Polyamide, Polyester, Polyacrylate, Polyvinylpyrrolidone, Polyurethane, Nafion, Styrol und Styrolderivate, funktionalisiertes Styrol wie beispielsweise Amberlyst-Materialien, Teflon und andere dem Fachmann bekannte Polymere, Co-polymere und modifizierte Polymere, sowie Kompositmaterialien aus Polymeren und anderen Festkörpern wie etwa Oxiden. Insbesondere sind Polymere bevorzugt, die polar sind, die Polarität kann sowohl intrinsisch über polare Gruppen induziert sein, als auch über nachträgliche Modifikation über dem Fachmann bekannte Methoden wir Oxidation.

Kohlenstoffhaltige Trägermaterialien können unter anderem sein: Aktivkohlen auf Basis von pflanzlichen, tierischen, mineralischen oder petrochemischen Ausgangsstoffen. Diese Aktivkohlen können unterschiedliche Anteile an graphitischem, hochgeordnetem und amorphem Kohlenstoff enthalten. Ebenso im Rahmen der Erfindung mit eingeschlossen sind unter anderem natürlicher oder synthetischer Graphit, synthetische Grafite können auf Basis von Braunkohle, Steinkohle, Erdöl, Erdölprodukten wie Schwerölen oder Destillationsrückständen oder Teeren, oder auch auf Basis von Kunststoffen wie Polyacrylnitril, ebenso wie hochgeordneter synthetischer Graphit.

Ebenso mit eingeschlossen sind Träger, die auf Kompositverbindungen aus Kohlenstoff und anderen Materialien basieren, wie beispielsweise Oxiden oder Metallen. Bei den kohlenstoffhaltigen Kompositverbindungen handelt es sich um Träger, die in einer Ausführungsform eingesetzt werden, die besonders bevorzugt ist. In dem Fall der kohlenstoffhaltigen Kompositmaterialien wird das Kompositmaterial aus dem kohlenstoffhaltigen Trägermaterial und dem anderen Material im Rahmen der Erfindung als ein einheitliches Trägermaterial betrachtet und fallen im Rahmen der Erfindung unter den Begriff der kohlenstoffhaltigen Träger. Dabei kann der Gewichtsanteil an Kohlenstoff in dem Kompositmaterial im Bereich von 1 - 99 Gew.-% betragen, bevorzugt von 10 - 90 Gew.-% betragen, besonders bevorzugt von 20 - 80 Gew.-%. Die anderen Materialien können unter anderem sein: Oxide der Haupt- und Nebengruppenelemente, Metalle, Nitride, Boride, Carbide.

Insbesondere mit eingeschlossen sind Träger auf Basis von Kompositverbindungen aus Kohlenstoff und anderen Materialien, bei denen der Kohlenstoff das andere Material umhüllend umgibt. Solche Träger können unter anderem sein: mit Kohlenstoff umhüllte Oxide wie beispielsweise SiO₂, TiO₂, ZrO₂ und/oder Al₂O₃, mit Kohlenstoff umhüllte Metalle, Nitride, Carbide und Boride. Solche Umhüllungen mit Kohlenstoff können durch dem Fachmann bekannte Methoden erfolgen. Als Beispiel ist hier das Aufbringen von Kohlenhydraten auf den jeweiligen Grundmaterialien und deren anschließende thermische Behandlung unter Stickstoff bei erhöhten Temperaturen zu nennen. Die Behandlung führt zur Wasserabspaltung und zur Ausbildung von einer schichtartigen Kohlenstoffumhüllung um die Grundmaterialien. Als Grundmaterialien können beispielsweise Oxide eingesetzt werden, insbesondere bevorzugt ist die Verwendung von SiO₂ als Grundmaterial.

Ebenfalls eingeschlossen sind Träger auf Basis von Carbiden von Metallen oder Nichtmetallen, wie beispielsweise Carbiden des Siliziums, insbesondere auch den verschiedenen Polytypen des Siliziumcarbids, bevorzugt sind insbesondere auch Siliziumcarbide mit spezifischen Oberflächen, die signifikant oberhalb der geometrischen Oberfläche der Partikel liegen. Dabei sind insbesondere solche Siliziumcarbide bevorzugt, die offenporös sind. Diese können sowohl silikatisch, als auch Nitrid- oder Oxynitrid gebunden sein, als auch über Rekristallisation gebunden. Insbesondere sind auch Siliziumcarbide miteingeschlossen, die so oberflächenbehandelt sind, dass sich eine Schicht von Siliziumdioxid auch der Oberfläche ausbildet. Solche Oberflächenbehandlungen können beispielsweise durch hydrothermale oder oxidative Behandlung erreicht werden. Eingeschlossen als Träger sind ebenfalls insbesondere die Carbide der Elemente Bor, Titan, Zirkon, Hafnium, Vanadium, Wolfram, Chrom, Molybdän, Tantal, Niob, Kobalt, Nickel und Eisen. Ebenfalls mit eingeschlossen sind Mischungen aus Carbiden, die Mischungen können sowohl als physikalische Mischungen oder Mischkristalle vorliegen.

Auch sind oberflächenbehandelte Carbide, die eine Oxidschicht ausbilden, miteingeschlossen. Auch Komposite aus Carbiden und kohlenstoffhaltigen Materialien sind als Träger miteingeschlossen.

Ebenfalls eingeschlossen sind Träger auf Basis von Nitriden von Metallen und/oder Nichtmetallen, wie beispielsweise Nitriden des Siliziums, bevorzugt sind insbesondere auch Siliziumnitride mit spezifischen Oberflächen, die signifikant oberhalb der geometrischen Oberfläche der Partikel liegen. Insbesondere sind auch Siliziumnitride miteingeschlossen, die so oberflächenbehandelt sind, dass sich eine Schicht von Siliziumdioxid auch der Oberfläche ausbildet. Solche Oberflächenbehandlungen können beispielsweise durch hydrothermale oder oxidative Behandlung erreicht werden. Eingeschlossen als Träger sind ebenfalls insbesondere die Nitride der Elemente Bor, Titan, Zirkon, Hafnium, Vanadium, Wolfram, Chrom, Molybdän, Tantal und Niob. Ebenfalls mit eingeschlossen sind Mischungen aus Nitriden, die Mischungen können sowohl als physikalische Mischungen oder Mischkristalle vorliegen.

Auch sind oberflächenbehandelte Nitride, die eine Oxidschicht ausbilden, in die Gruppe der Träger miteingeschlossen. Auch Komposite aus Nitriden und kohlenstoffhaltigen Materialien sind in die Gruppe der Träger miteingeschlossen.

Ebenfalls eingeschlossen sind Träger auf Basis von Boriden von Metallen oder Nichtmetallen. Bevorzugt sind insbesondere auch Boride mit spezifischen Oberflächen, die signifikant oberhalb der geometrischen Oberfläche der Partikel liegen. Signifikant bedeutet, dass die Oberfläche mindestens um den Faktor 1,5 größer ist als die geometrische Oberfläche. Weiter vorzugsweise ist die Oberfläche mindestens um den Faktor 2 größer als die geometrische Oberfläche. Insbesondere sind auch Boride miteingeschlossen, die so oberflächenbehandelt sind, dass sich eine Schicht von Oxid auf der Oberfläche ausbildet. Solche Oberflächenbehandlungen können beispielsweise durch hydrothermale oder oxidative Behandlung erreicht werden. Eingeschlossen in die Gruppe der Träger sind ebenfalls insbesondere die Boride der Elemente Titan, Zirkon, Hafnium, Vanadium, Wolfram, Chrom, Molybdän, Tantal, Nickel, Kobalt, Eisen und Niobium. Ebenfalls mit eingeschlossen sind Mischungen aus Boriden, wobei die Mischungen sowohl als physikalische Mischungen als auch als Mischkristalle vorliegen können.

Auch sind diejenigen oberflächenbehandelte Boride, die eine Oxidschicht ausbilden, in die Gruppe der Trägereingeschlossen. Auch zählen Komposite aus Boriden und kohlenstoffhaltigen Materialien zur Gruppe der Träger.

Ebenfalls mit eingeschlossen in die Gruppe der Träger sind oxidische Träger. Solche oxidischen Träger können als binäre Verbindungen vorliegen, also als Verbindung aus einem Haupt- oder einem Nebengruppenelement und Sauerstoff, oder auch ternär - also als Verbindung aus zwei Hauptgruppenelementen mit Sauerstoff oder einem Hauptgruppenelement gemeinsam mit einem Nebengruppenelement und Sauerstoff. Die Träger können auch als multinäre oxidische Verbindungen vorliegen, also als Verbindung, die mehr als zwei Haupt- und/oder Nebengruppenelemente und Sauerstoff enthalten. Als Haupt- oder Nebengruppenelemente kommen alle Elemente des Periodensystems außer den Edelgasen in Frage.

Beispiele hierfür sind unter anderem SiO₂ in den dem Fachmann bekannten Modifikationen, Al₂O₃ in den dem Fachmann bekannten Modifikationen, TiO₂ in den dem Fachmann bekannten Modifikationen, ZrO₂ in den dem Fachmann bekannten Modifikationen. Ebenfalls unter diesen Punkt fallen binäre Oxide, in denen das Haupt- oder Nebengruppenelement in mehr als einer Oxidationsstufe vorliegt, wie beispielsweise Mn₃O₄, Fe₃O₄ oder andere gemischtvalente Oxide. Die Träger können kristallin oder röntgenamorph vorliegen. Die oxidischen Träger können auch polynäre Oxide enthalten, die aus mehr als einem Haupt- und/oder Nebengruppenelement und Sauerstoff aufgebaut sind und die entweder in einer gemeinsamen kristallinen Verbindung oder in Kombination mit röntgenamorphen Verbindungen vorliegen. Beispiele hierfür sind MgAl₂O₄, LaAlO₃, CaTiO₃, CeZrO₄ H₂Al₁₄Ca₁₂O₃₄. Ebenfalls mit eingeschlossen sind Mischungen aus zwei oder mehr binären und/oder polynären Oxiden, die als sogenannte gemischte Oxide vorliegen und die dabei entweder in unterschiedlichen oder in vereinigten Mischungen von kristallinen und/oder röntgenamorphen Verbindungen vorliegen. Ebenfalls mit eingeschlossen sind zeolithische Träger, mikroporöse Molekularsiebe auf Basis von Alumophosphaten sowie organische poröse Gerüstverbindungen- wie beispielsweise die sogenannten MOF-Verbindungen. Bei zeolithischen Trägern handelt es sich um Träger, die zumindest einen Zeolithen umfassen. Ebenfalls mit eingeschlossen in die Gruppe der Träger sind salz- oder glasartige Verbindungen mit komplexen Oxoanionen, wie Borate, Aluminate, Carbonate, Silicate, Stanate, Nitrate, Phosphate, Antimonate, Arsenate, Oxalate, Sulfate, Selenate, Tellurate, Vanadate, Chromate, Manganate und andere dem Fachmann bekannte salz- oder glasartige Verbindungen mit komplexen Oxoanionen. Ebenfalls mit eingeschlossen sind binäre und polynäre Hydroxide wie Hydrotalcite. Ebenfalls mit eingeschlossen sind die Salze von Haupt- und/oder Nebengruppenelementkomplexen wie Fe₄[Fe(CN)₆]₃. Ebenfalls mit eingeschlossen sind Träger mit Haupt- und/oder Nebengruppenelemente der Zyanide, Zyanate und Isozyanate.

Der Träger kann als Pulver, Dispersion, Kolloid, Granulat und/oder als Formkörper eingesetzt werden. In einer besonderen Ausführungsform wird der Träger als Formkörper eingesetzt. Solche Formkörper können in Form von Tabletten, als unregelmäßiger Bruch, Extrudate, Kugeln oder anderen dem Fachmann bekannten Formkörpern eingesetzt werden. Die hier dargestellte Ausgestaltung der Formkörper bezieht sich ebenso auf diejenigen Ausführungsformen, in denen der erfindungsgemäße Katalysator als Vollkatalysator vorliegt.

In einer bevorzugten Ausführungsform ist der Katalysator mit der katalytisch aktiven Komponente für das Verfahren zur Herstellung von C₃- bis C₁₉-Aldehyden und/oder Alkoholen dadurch gekennzeichnet, dass dieser als Vollkatalysator und/oder als Trägerkatalysator ausgestaltet vorliegt, wobei der Katalysator vorzugsweise als Trägerkatalysator ausgestaltet ist Vorzugsweise ist die katalytisch aktive Komponente in Kontakt mit dem Träger und wird durch den Träger fixiert, weiter vorzugsweise weist der Trägerkatalysator einen Gewichtsanteil an katalytisch aktiver Komponente auf, der in Bezug auf die Summe aus katalytisch aktiver Komponente und Träger im Bereich von 0,01 - 99,5 % liegt, vorzugsweise liegt der Gewichtsanteil an katalytisch aktiver Komponente in Bezug auf die Summe an katalytisch aktiver Komponente und Träger im Bereich von 0,1 - 50 %, darüber hinaus bevorzugt liegt der Gewichtsanteil an katalytisch aktiver Komponente in Bezug auf die Summe an katalytisch aktiver Komponente und Träger im Bereich von 0,5 - 10 %. Sofern der Katalysator mit Promotoren oder Dotierungen versehen ist, so werden diese bei dieser Mengenangabe dem Gewichtsanteil an Träger zugerechnete. Somit bedeutet dann bei dieser Angabe, dass der Gewichtsanteil an Träger durch die Summe aus Träger, Dotierung und Promotor gebildet wird. Bei dieser Angabe der Menge der katalytisch aktiven Komponente sind dem Träger somit auch noch weitere Bestandteile zuzurechnen, die im Katalysator enthalten sind, jedoch nicht die katalytisch aktive Komponente darstellen. Dadurch wird auch klar, dass geringe Mengen an katalytisch aktiver Menge ausreichen, um das erfindungsgemäße Verfahren durchführen zu können.

Von Bedeutung für die Erfindung ist, dass die eingesetzten Träger eine ausreichend hohe BET-Oberfläche aufweisen. Bevorzugt weisen die Träger eine BET-Oberfläche auf, die im Bereich von 1 bis 1000 m²/g liegt, besonders bevorzugt liegt die BET-Oberfläche im Bereich von 5 bis 500 m²/g, ganz besonders bevorzugt liegt die BET-Oberfläche im Bereich von 10 bis 250 m²/g. Die Angabe der BET-Oberfläche bezieht sich auf die Bestimmung der Oberfläche mittels Stickstoffadsorption gemäß DIN-Norm 66131 (Juli 1993).

Durch die Modifikation des Trägers mit der phosphidhaltigen Komponente kann sich die spezifische Oberfläche des finalen Trägerkatalysators im Vergleich zu dem eingesetzten Träger verändern. Bei einem Vergleich der BET-Oberfläche des eingesetzten Trägers mit der BET-Oberfläche des fertigen Trägerkatalysators, kann diese entweder größer oder kleiner werden.

Wird der Katalysator als Voll- oder Trägerkatalysator eingesetzt, ist ebenfalls eine geeignete spezifische Oberfläche vorteilhaft. Bevorzugt liegt diese spezifische Oberfläche, die durch die BET-Methode bestimmt wird, im Bereich von 1 bis 1000 m²/g, besonders bevorzugt im Bereich von 5 bis 250 m²/g, ganz besonders bevorzugt im Bereich von 10 bis 100 m²/g.

In einer bevorzugten Ausführungsform weist der erfindungsgemäße Katalysator mit der katalytisch aktiven Komponente zur Herstellung von C₃- bis C₁₉-Aldehyden und/oder Alkoholen eine BET-Oberfläche auf, die in einem Bereich von 1- 1000 m²/g liegt, weiter bevorzugt liegt die BET-Oberfläche im Bereich von 5 - 250 m²/g, und insbesondere bevorzugt im Bereich von 10 - 100 m²/g.
Für eine Reihe der erfindungsgemäßen Reaktionen ist ein optimierter Stofftransport innerhalb des jeweiligen Katalysatorkornes von Bedeutung, um hohe intrinsische Aktivitäten und hohe spezifische Selektivitäten hinsichtlich der Hydroformylierung als der Zielreaktion beziehungsweise der Herstellung von C₃- bis C₁₉-Aldehyd und/oder Alkloholen als Zielprodukten zu erreichen. Das Design des Porensystems ist daher ein elementarer Bestandteil des Katalysatordesigns. Porensysteme eines Katalysatorkornes können Poren von unterschiedlichen Radien besitzen, dabei werden diese Porenradien durch unterschiedliche Methoden bestimmt. Die Bestimmung der Porenradienverteilung erfolgt dabei über die für den jeweiligen Porenradienbereich geeigneten Methode: Makro- und Mesoporenradien und -verteilung werden über Quecksilberporosimetrie bestimmt, Mikro- und Mesoporenradien und -verteilung werden über Stickstoffadsorbtion bestimmt. Die jeweiligen Methoden und Methoden der Auswertung der erhaltenen Daten sind dem Fachmann bekannt und als standardisierte Methoden vorgegeben.

Zur Bestimmung der spezifischen Oberfläche von Feststoffen durch Gasadsorption nach dem BET-Verfahren ist hier beispielsweise auf die DIN ISO 9277: 2003-05 verwiesen. Zur Bestimmung der Porenradienverteilung mittels der BJH-Methode ist hier auf die ISO 9277: 1995 verwiesen. Weitere Referenzen sind ISO 9277: 1995; zum Beispiel die Bestimmung der Porenradienverteilung mittels der BJH Methode BJH-Methode, DIN 66134; oder zum Beispiel: die Norm ISO 15901-1 und DIN 66133. Eine Grundlage des Messverfahrens der Quecksilberintrusion ist das Verhalten nichtbenetzender Flüssigkeiten in Kapillaren. Näheres zur Mesoporenanalyse nach BJH (einem Verfahren zur Auswertung von Gasadsorptionsuntersuchungen) siehe DIN 66134. Näheres zur Quecksilberporosimetrie siehe DIN 66133.

In einer bevorzugten Ausführungsform weisen die Katalysatoren, die als Trägerkatalysatoren oder als Vollkatalysatoren vorliegen, eine Porenstruktur auf, in der auch Mikroporen oder Mesoporen beziehungsweise Mikorporen und Mesoporen enthalten sind. Darüber hinaus können die Katalysatoren auch Makroporen enthalten. In Bezug auf die Mikroporen beziehungsweise auch in Bezug auf die Mesoporen lässt sich sagen, dass diese als monomodale Poren vorliegen, die jeweils einen einheitlichen Porendurchmesser in Bezug auf die Mikroporen und die Mesoporen aufweisen können.
Somit lässt sich hinsichtlich der Dimensionierung der Poren der Vollkatalysatoren beziehungsweise der Trägerkatalysatoren feststellen, dass diese Mikroporen und/oder Mesoporen aufweisen und diese jeweils als monomodales oder multimodales Porensystem ausgestaltet sein können. Darüber hinaus umfasst die Erfindung auch Ausführungsformen von Katalysatoren, die eine mono- oder multimodale Strukturierung der jeweiligen Gruppe von Poren der Markro-, Meso- und/oder Mikroporen aufweisen.
In einer bevorzugten Ausführungsform enthalten die Katalysatoren zumindest einen der nachfolgend genannten Träger: kohlenstoffhaltige Träger, kohlenstoffmodifiziertes SiO₂, SiC beziehungsweise oxidativ oder hydrothermal modifiziertes SiC.

### II. Verfahren zur Herstellung des Katalysators

Das Herstellungsverfahren des Katalysators ist durch die folgenden Schritte gekennzeichnet:
(i) Vereinigung von Phosphorquelle, Quelle für Verbindung, die mit Phosphorquelle reagiert, und Träger, vorzugsweise umfasst die Vereinigung die Zuführung eines Lösungsmittels,
(ii) thermische Behandlung,
(iii) reduzierende Behandlung mit Reduktionsmittel,
sowie optional: (iv) oxidierende Behandlung;
wobei die Verfahrensschritten (i), (ii), (iii) auch durch die Verfahrensschritte (i'), (iii') ersetzt werden können:
(i') Vereinigung von Träger mit der Quelle der Verbindung und/oder Verbindungen, die mit Phosphorquelle reagiert, und Überführung des vereinigten Gemisches, das einen Träger mit Nanopartikeln aus Metallen und/oder Metallverbindungen beziehungsweise Elementen bildet,
(iii') reduzierende Behandlung, wobei der Träger mit den Nanopartikeln vor oder während der reduzierenden Behandlung mit einer Phosphorquelle kontaktiert wird beziehungsweise die reduzierende Behandlung mit einer Phosphorquelle vorgenommen wird, wobei der Katalysator für die Herstellung von C₃- bis C₁₉-Aldehyden und/oder Alkoholen, bei dem als katalytisch aktive Komponente Rh- oder Co-Phosphid oder Gemische der beiden, vorzugsweise Rh-Phosphid, verwendet wird.

Bezüglich des Verfahrens zur Herstellung des erfindungsgemäßen Katalysators gibt es unterschiedliche Verfahrensvarianten, wobei die Darstellung sich zunächst auf ein Verfahren bezieht, das die Verfahrensschritte (i), (ii) und (iii) umfasst. Die vorliegend gegebene Darstellung des Verfahrens zur Herstellung des erfindungsgemäßen Katalysators ist weder abschließend noch einschränkend. Bei den hier aufgeführten Verfahrensvarianten handelt es sich um Ausführungsformen, die besonders bevorzugt sind.

Zunächst erfolgt die Erläuterung des erfindungsgemäßen Verfahrens zur Herstellung des Katalysators, welches die Verfahrensschritte (i) - (iii) umfasst. In Bezug auf die Art und Weise, wie die Vereinigung der Ausgangsquellen durchgeführt wird, sind Abwandlungen möglich, wie im nachfolgenden Teil näher dargestellt.

In den einzelnen Ausführungsformen des erfindungsgemäßen Verfahrens zur Herstellung der Katalysatormaterialien können die einzelnen Teilschritte beziehungsweise die Verfahrensschritte durch die nachfolgend aufgeführten Merkmale gekennzeichnet sein: Herstellung durch Kombination der Elemente bei erhöhter Temperatur, Herstellung durch Festkörper-Metathese wie beispielsweise die Umsetzung von Alkalimetallphosphiden mit wasserfreien Chloriden, Umsetzung von Phosphan mit Metallchloriden, Umsetzung von Organometallverbindungen mit Organophosphorverbindungen bei erhöhter Temperatur, Elektrolyse von salzartigen Verbindungen des Phosphors mit geeigneten Metall-Präkursoren, Reduktion von Orthophosphaten, Oxidphosphaten, Pyrophosphaten, Oligophosphaten, Polyphosphaten, Metaphosphaten oder Ultraphosphaten, sowie phosphaten mit heteroatomen als Teil des Phosphatnetzwerks wie Silicophosphaten oder Phosphatsilicophosphaten. Abscheidung gasphasenlöslicher Spezies im Sinne eines chemischen oder physikalischen Gasphasentransports.

In einer bevorzugten Ausführungsform bezieht sich die Erfindung auch auf ein Verfahren zur Herstellung eines Katalysatorvorläufermaterials. Bei dem Verfahren zur Herstellung des Katalysatorvorläufermaterials werden gemäß dem Verfahrensschritt (i) eines oder mehrere der Elemente beziehungsweise Verbindungen der Elemente aus der Gruppe Co, Rh mit einer Phosphorquelle in Kontakt gebracht und anschließend gemäß des Verfahrensschritts (ii) einer thermischen Behandlung unterzogen, die zu einer Bildung eines kristallinen oder amorphen Phosphates führt. Die thermische Behandlung wird dabei besonders bevorzugt bei Temperaturen im Bereich von 25 - 800 °C durchgeführt, besonders bevorzugt bei Temperaturen im Bereich von 50 - 600 °C durchgeführt, ganz besonders bevorzugt bei Temperaturen im Bereich von 100 - 500 °C durchgeführt.

Die Art der Gasatmosphäre bei der thermischen Behandlung des Vorläufermateriales gemäß des Verfahrensschritts (ii) ist vorzugsweise oxidierend oder inert. Geeignete Gase sind unter anderem Luft, Mischungen von Stickstoff und Sauerstoff, Stickstoff, Kohlendioxid, Wasserdampf in Verdünnung in Luft oder Stickstoff, Stickoxide, Lachgas und andere dem Fachmann bekannte oxidierende oder nicht oxidierende Gase. Auch Mischungen der hier aufgeführten Gase können verwendet werden.

Das Katalysatorvorläufermaterial kann als geträgertes Material oder als Vollmaterial vorliegen. Besonders bevorzugt im Sinne der Erfindung ist es, wenn das Katalysatorvorläufermaterial als geträgertes Material vorliegt.

Als Phosphorquellen zur Herstellung des Katalysatorvorläufermateriales können unter anderem die folgenden Verbindungen eingesetzt werden: Phosphorsäure, phosphorige Säure, Di-, Meta-, Poly-Phosphorsäure, Ammoniumphosphate, Ester der Phosphorsäure, Alkaliphosphate, P₂O₅. Besonders bevorzugt werden Phosphorquellen zur Herstellung des Katalysatorvorläufermateriales eingesetzt, die in Wasser und/oder in organischen Lösungsmitteln löslich sind, vorzugsweise sind die Phosphorquellen in Mischungen aus Wasser und organischen Lösungsmitteln löslich. Ganz besonders bevorzugt werden Phosphorquellen zur Herstellung des Katalysatorvorläufermateriales eingesetzt, die in Wasser löslich sind.

Weitere Phosphorquellen, die hier zum Einsatz kommen können sind Phosphan, organo-Phosphorverbindungen mit Phosphor-Kohlenstoff-Bindungen, hochsiedende organische Phosphorverbindungen wie Tri-octyl-Phosphor-ether, elementarer Phosphor in allen dem Fachmann bekannten Modifikationen - sowohl in fester als auch in gelöster oder dispergierter Form, Phosphortrichlorid und andere dem Fachmann bekannte Phosphorverbindungen, die reduzierend wirken können. Zusätzlich zu solchen reduzierend wirkenden Phosphorverbindungen können alternative Reduktionsmittel in einem oder mehreren Schritten zum Einsatz kommen.

Die Phosphorquelle wird mit einem Präkursor beziehungsweise einer Präkursorverbindung zur Reaktion gebracht. Die Präkursoren sind somit die Reaktionspartner der Phosphorquelle. Die Präkursoren der Elemente aus der Gruppe Co, Rh können als Nitrate, Chloride, Bromide, Sulfate, Sulfite, Hydroxide, Formiate, Acetate, Alkoholate, Komplexverbindungen, Organometallverbindungen, Acetylacetonate, Carbonylverbindungen, Oxalate, Carbonate, basische Carbonate, Hydroxyde, Oxide und andere dem Fachmann bekannte geeignete Präkursoren der genannten Elemente, diese werden im Rahmen der Erfindung auch als Metallverbindungen bezeichnet. Besonders bevorzugt sind die Präkursoren zur Herstellung des Katalysatorvorläufermateriales in Wasser, in organischen Lösungsmitteln und/oder Mischungen aus Wasser und organischen Lösungsmitteln löslich, ganz besonders bevorzugt sind die Präkursoren zur Herstellung des Katalysatorvorläufermateriales in Wasser löslich. Bei den Lösungen kann es sich um kolloidale oder echte Lösungen der Molekülverbindungen oder Salze handeln. Bei den kolloidalen Lösungen kann es sich auch um kolloidale Lösungen von Metall- und/oder Metalloxid-Nanopartikeln handeln. Besonders bevorzugt werden hierbei diejenigen Verbindungen beziehungsweise Metalle als Präkursoren verwendet, die zumindest ein Metall aus der Gruppe Co, Rh enthalten, vorzugsweise Rh.

Das Katalysatorvorläufermaterial enthält in der Regel einen Phosphorüberschuss bezogen auf die Stöchiometrie der kristallinen Phosphide der Elemente aus der Gruppe Co, Rh.

Die jeweiligen binären kristallinen Phosphide, auf die sich die Stöchiometrie bezieht, sind unter anderem: Co₂P, CoP, CoP₃ Rh₂P, Rh₁₂P₇, Rh₃P₂, Rh₄P₃, RhP₂, RhP₃. Da die Stöchiometrie der Verbindungen der sehr stark von der jeweiligen Zielphase des kristallinen Phosphids abhängt, ist eine Anpassung der angebotenen Phosphormenge notwendig. Die in dem Verfahren eingesetzte Phosphormenge wird in der Regel so gewählt, dass eventuell entstehende Verluste bei der späteren reduktiven Behandlung des Katalysatorvorläufermateriales durch Bildung flüchtigen Phosphorkomponenten wie beispielsweise von Phosphan (PH₃) kompensiert werden. Vorzugsweise liegt der Überschuss an bei dem Verfahren eingesetztem Phosphor bezogen auf das aus dem Verfahren resultierende Phosphid und die dadurch bedingte Stöchiometrie bezogen auf den Phosphorgehalt in einem Bereich von 0,5 - 300 % auf molarer Basis, besonders bevorzugt in einem Bereich von 0,75 - 200 % auf molarer Basis, ganz besonders bevorzugt in einem Bereich von 1 - 100 % auf molarer Basis.

Sofern die Vereinigung der Ausgangsquellen unter Schritt (i) auch ein Lösungsmittel umfasst, so wird dieses vorzugsweise vor der thermischen Behandlung, d.h. dem Schritt (ii), entfernt. Die Entfernung eines Lösungsmittels durch thermische Behandlung wird im Rahmen der Erfindung als Trocknung bezeichnet. Die Entfernung des Lösungsmittels nach Vereinigung der Präkursoren beziehungsweise Ausgangsquellen kann durch Filtration und durch thermische Behandlung erfolgen. In einer Reihe von Fällen kann die Filtration entfallen, so beispielsweise bei der Herstellung eines geträgerten Katalysators, insbesondere dann, wenn der Träger in Pulverform, als Granulat oder als Formkörper vorliegt. Geeignete Methoden sind unter anderem Gefriertrocknung, Sprühtrocknung, Trocknung im Hordenofen unter erhöhter Temperatur, Trocknung im Drehrohrofen unter erhöhter Temperatur und andere dem Fachmann bekannte Trocknungsmethoden.

Die Trocknung findet dabei bevorzugt bei Temperaturen im Bereich von -60 - 600 °C statt, besonders bevorzugt bei Temperaturen im Bereich von -20 - 500 °C statt, ganz besonders bevorzugt bei Temperaturen im Bereich von -10 - 400 °C statt.

Nachfolgend wird das erfindungsgemäße Verfahren in mehreren Varianten näher spezifiziert, wobei der Schritt (i) des Verfahrens in die Teilschritte (i.2) - (i.5) beziehungsweise in die Teilschritte (i.1) - (i.5) untergliedert wird. Der Teilschritt (i.1) umfasst das Vorlegen eines Trägers. Bei einem Verzicht auf den Teilschritt (i.1) führt dies zu einem Verfahren, durch welches ein Vollkatalysator hergestellt wird.

Das Verfahren zu Herstellung des Katalysatorvorläufermateriales beziehungsweise des Katalysators als Vollkatalysator erfolgt dabei nach den folgenden Schritten:
(i.2) Vorlegen eines Präkursors der Elemente aus der Gruppe Co, Rh, vorzugsweise Rh, in einem Lösungsmittel,
(i.3) Vorlegen einer oder mehrerer Phosphorquellen in einem Lösungsmittel,
(i.4) Vereinigen des Präkursors der der Elemente aus der Gruppe Co, Rh, vorzugsweise Rh, mit der einen oder mehreren Phosphorquellen,
(i.5) Entfernen des Lösungsmittels,
(ii.1) Durchführung einer thermischen Behandlung,
wobei die Durchführung der Schritte (i.2) und (i.3) nicht an eine Reihenfolge gebunden ist.

Dabei ist anzumerken, dass in einer besonders bevorzugten Ausführungsform des Verfahrens zur Herstellung in den Schritten (i.2) und (i.4) die Präkursoren der Elemente aus der Gruppe Rh, Co, vorzugsweise Rh, eingesetzt werden.

Die Zugabe von Dotierelementen erfolgt vorzugsweise in Verbindung mit einem der Schritte (i.2), (i.3) und/oder (i.4). Im Anschluss daran erfolgt Schritt (iii), der eine reduzierende Behandlung des Katalysatorvorläufermaterials beziehungsweise des Katalysators mit einem Reduktionsmittel umfasst.

Das Verfahren zur Herstellung des Katalysatorvorläufermateriales beziehungsweise des Katalysators als Trägerkatalysator erfolgt vorzugsweise nach den folgenden Schritten:
(i.1) Vorlegen eines Trägers,
(i.2) Vorlegen eines Präkursors der Elemente aus der Gruppe Co, Rh, vorzugsweise Rh, in einem Lösungsmittel,
(i.3) Vorlegen einer oder mehrerer Phosphorquellen in einem Lösungsmittel,
(i.4) Vereinigen des Präkursors der der Elemente aus der Gruppe Co, Rh, vorzugsweise Rh, mit der einen oder mehreren Phosphorquellen und des Trägers in mindestens einem Schritt,
(i.5) Entfernen des Lösungsmittels,
(ii) Durchführung einer thermischen Behandlung.

Die Zugabe von Dotierelementen kann mit den Schritten (i.1), (i.2), (i.3) und/oder (i.4) erfolgen. Die Reihenfolge bei der Durchführung der einzelnen Verfahrensschritte (i.1), (i.2), (i.3) und/oder (i.4) kann alterniert werden.

Wobei der Teilschritt (i.4) so ausgeführt werden kann, dass sowohl der Präkursor aus Schritt (i.2) als auch die Phosphorquelle aus Schritt (i.3) in einem ersten Schritt mit dem Träger vereinigt werden können.
(iii) Reduzierende Behandlung mit einem Reduktionsmittel.
Sowie optional: (iv) oxidierende Behandlung.

Die Zugabe von Dotierelementen kann in Verbindung mit den jeweiligen Teilschritten (i.1), (i.2), (i.3) und/oder (i.4) erfolgen. Darüber hinaus ist die Reihenfolge bei der Vornahme der einzelnen Teilschritte variabel.

Wobei der Teilschritt (i.4) so ausgeführt werden kann, dass sowohl der Präkursor aus Teilschritt (i.2) als auch die Phosphorquelle aus Teilschritt (i.3) in einem ersten, zusammenhängenden Schritt mit dem Träger vereinigt werden können.

In einer Verfahrensvariante kann der Teilschritt (i.3) vor dem Teilschritt (i.2) ausgeführt werden.

In einer weiteren Verfahrensvariante können nach dem Teilschritt (i.2) die Schritte (i.5) und (ii) und dann der Schritt (i.3) gefolgt von (i.4), (i.5) und (ii) ausgeführt werden.

Danach kann optional ein Schritt analog (i.5) erfolgen, dem dann die Vereinigung mit dem oder den anderen jeweiligen Präkursoren erfolgt. Im Anschluss an die thermische Behandlung gemäß des Schritt (ii) erfolgt der Schritt (iii).

Das Katalysatorvorläufermaterial wird in das Katalysatormaterial überführt beziehungsweise in dieses umgewandelt, das zur Durchführung des erfindungsgemäßen Verfahrens eingesetzt wird. Die Umwandlung erfolgt durch einen thermischen Behandlungsschritt in reduzierender Atmosphäre gemäß dem Schritt (iii). In einer Ausführungsform des Verfahrens können der Schritt (ii) und der Schritt (iii) zu einem Schritt (ii)+(iii) kombiniert werden. Dabei können die folgenden Gase als Reduktionsmittel zur Anwendung kommen: Wasserstoff, Synthesegas in variablen Zusammensetzungen, Mischungen von Kohlendioxid und Wasserstoff in variabler Zusammensetzung, Formiergas, Mischungen von Stickstoff und Wasserstoff in variabler Zusammensetzung und weitere dem Fachmann bekannte Gase und verdampfbare Flüssigkeiten, die reduzierend wirken. Auch die Gegenwart von Wasserdampf in die Gasatmosphäre bei der Behandlung ist in einer Ausführungsform des Verfahrens eingeschlossen. Die bevorzugte Temperatur des thermischen Behandlungsschrittes in reduzierender Atmosphäre liegt bevorzugt im Bereich von 50 - 1200 °C, besonders bevorzugt im Bereich von 80 - 1000 °C, ganz besonders bevorzugt im Bereich von 100 - 800 °C.

In einer bevorzugten Ausführungsform umfasst das Verfahren zur Herstellung des Katalysators auch einen Schritt (iv), der optional ist: Um die Reaktivität des Katalysatormaterialies gegenüber Luft nach dem thermischen Behandlungsschritt in reduktiver Atmosphäre zu minimieren, kann ein sogenannter Passivierungsschritt erfolgen. Dieser wird vorzugsweise bei einer Temperatur im Bereich von 20 bis 500°C unter leicht oxidierender und/oder wasserhaltiger Atmosphäre durchgeführt. Bevorzugt ist eine Passivierung bei einer Temperatur im Bereich von 20 bis 500 °C, besonders bevorzugt ist eine Passivierung bei einer Temperatur im Bereich von 30 bis 400 °C, ganz besonders bevorzugt ist eine Passivierung bei einer Temperatur im Bereich von 50 bis 300 °C. Die bevorzugte Atomsphäre enthält zwischen 0,2 und 20 Vol.-% Sauerstoff in einem Inertgas, besonders bevorzugt sind 0,5 und 15 Vol.-% Sauerstoff in einem Inertgas, ganz besonders bevorzugt sind 0,7 und 10 Vol.-% Sauerstoff in einem Inertgas. Der Wassergehalt in der Sauerstoff/Inertgasmischung kann im Bereich von 1 - 20 Vol.-% betragen, bevorzugt von 2 - 15 Vol.-%, besonders bevorzugt von 3 - 10 Vol.-%. Andere Oxidationsmittel als Sauerstoff sind beispielsweise Ozon, Stickoxide, N₂O sowie H₂O₂, deren Verwendung auch Ausführungsformen des erfindungsgemäßen Verfahrens bildet.

In einer bevorzugten Ausführungsform erfolgt das Verfahren zur Herstellung des Katalysators als Trägerkatalysator in einer abgewandelten Form, wobei die Verfahrensschritte (i') und (iii') vorgenommen werden.

Verfahren zur Herstellung des Katalysatorvorläufermateriales beziehungsweise des Katalysators als Trägerkatalysator erfolgt vorzugsweise nach den folgenden Schritten:
Teilschritte von Schritt (i') sind:
(i.1) Vorlegen eines Trägers,
(i.2) Vorlegen eines Präkursors der Elemente aus der Gruppe Co, Rh, vorzugsweise Rh, in einem geeigneten Lösungsmittel,
(i.4) Vereinigen des Präkursors der Elemente aus der Gruppe Co, Rh, vorzugsweise Rh, mit dem Träger in mindestens einem Schritt,
(i.5) Entfernen des Lösungsmittels,
(ii) Durchführung einer thermischen Behandlung,
(iii') Reduzierende Behandlung mit einem Reduktionsmittel, vorzugsweise in Gegenwart einer Phosphorquelle,
sowie optional: (iv) oxidierende Behandlung.

Die thermische Behandlung und die Behandlung in reduzierender Atmosphäre können gleichzeitig vorgenommen werden, wodurch Schritt (ii) und Schritt (iii') zu Schritt (ii)+(iii') vereinigt werden. Die bevorzugten Bedingungen für die thermischen Behandlung unter Schritt (ii) beziehungsweise für den thermischen Behandlungsschritt in Gegenwart von einem Reduktionsmittel gemäß den zusammengefassten Schritten (ii)+(iii') waren bereits zuvor in der Beschreibung genannt worden.

Zumindest einer der Schritte (ii), (iii') oder (ii)+(iii') erfolgt in Gegenwart einer Phosphorquelle.

Der Verfahrensschritt (i') unterscheidet sich vom Verfahrensschritt (i) dahingehend, dass im Verfahrensschritt (i) der Teilschritt (i.3) nicht enthalten ist, so dass die Vereinigung des Trägers und des Präkursors gemäß des Teilschritts (i.4) in Abwesenheit von Phosphor erfolgt.

In der vorliegenden und abgewandelten Ausführungsform umfasst das Verfahren zur Herstellung des erfindungsgemäßen Katalysatormaterials beziehungsweise eines Katalysatorvorläufermaterials diejenigen Schritte, mittels denen zunächst auf einem Träger hochdisperse metallische Nanopartikel beziehungsweise Metallverbindungen in nanopartikulärer Form abgeschieden werden. Solche metallischen Nanopartikel beziehungsweise Metallverbindungen bilden hochreaktive Präkursoren, die in der Lage sind, mit der Phosphorquelle unter reduktiven Bedingungen zu Metallphosphiden zu reagieren. Somit erfolgt die reduzierende Behandlung gemäß dem Schritt (iii') in Gegenwart einer Phosphorquelle.

Die metallischen Nanopartikel beziehungsweise Verbindungen zur Herstellung des Katalysatorvorläufermateriales gemäß der abgewandelten Ausführungsform des Verfahrens haben bevorzugt eine Größe im Bereich von 1 bis 200 nm, besonders bevorzugt eine Größe im Bereich von 2 bis 100 nm, ganz besonders bevorzugt eine Größe im Bereich von 3 bis 50 nm.

Das Verfahren zur Herstellung der metallischen Nanopartikel auf einem Träger gemäß dem Schritt (i') kann über Tränkverfahren von geeigneten Präkursoren mit anschließender reduktiver Behandlung erfolgen. Geeignete Präkursoren sind unter anderem Nitrate, Chloride, Bromide, Sulfate, Sulfite, Formiate, Acetate, Alkoholate, Komplexverbindungen, Organometallverbindungen, Acetylacetonate, Carbonylverbindungen, Oxalate, Carbonate, basische Carbonate, Hydroxyde, Oxide und andere dem Fachmann bekannte geeignete Verbindungen.

Ebenfalls mit eingeschlossen sind Ausführungsformen, bei denen die Präkursoren im Schritt (i.2) in Form von kolloidalen Partikeln beziehungsweise in nanopartikulärer Form in Lösung vorgelegt werden. Die kolloidalen Partikel beziehungsweise die in nanopartikularer Form vorliegenden Partikel können vollständig aus metallischen Partikeln bestehen oder die Metalle zum Teil in ihrer oxidischen Form enthalten. Solche kolloidalen Lösungen sind dem Fachmann bekannt. Die Herstellung der kolloidalen Lösungen kann die Zugabe von Stabilisatoren umfassen, die in Form von molekularen und/oder polymeren Stabilisatoren zugesetzt werden. Als Grundlage für die Präkursorlösung wird vorzugsweise ein wässriges und/oder organisches Lösungsmittel eingesetzt, besonders bevorzugt ist jedoch die Verwendung eines wässrigen Lösungsmittels.

Gemäß des Schritts (i') werden im Teilschritt (i.1) ein Träger vorgelegt, im Teilschritt (i.2) der oder die Präkursoren vorgelegt und im Teilschritt (i.4) die vorgelegten Ausgangsquellen (mit Ausnahme der Phosphorquelle) vereinigt.

Gegebenenfalls kann nach dem nach dem Inkontaktbringen des Trägers mit dem im Solvens gelösten Präkursor dieses durch einen geeigneten Teilschritt (i.5) entfernt werden. In einer bevorzugten Ausführungsform erfolgt dieser Schritt vor der Behandlung mit einem flüssigen, gelösten und/oder gasförmigen Reduktionsmittel gemäß des Schritts (iii'). Die Entfernung des Solvens kann dabei durch eine Trocknungsbehandlung (beziehungsweise eine thermische Behandlung gemäß des Schritts (ii)) erfolgen. Die geeigneten Parameter für die Durchführung der Trocknungsbehandlung beziehungsweise der thermischen Behandlung waren bereits zuvor in der vorliegenden Beschreibung genannt worden. Geeignete Methoden sind unter anderem Gefriertrocknung, Sprühtrocknung, Trocknung im Hordenofen unter erhöhter Temperatur, Trocknung im Drehrohr unter erhöhter Temperatur und anderen dem Fachmann bekannte Trocknungsmethoden.

Die reduktive Behandlung des Katalysatorvorläufermateriales gemäß Schritt (iii') des Herstellungsverfahrens in der abgewandelten Ausführungsform kann dabei mit flüssigen und/oder gelösten Reduktionsmitteln erfolgen. Beispiele hierfür sind Natriumborhydrid, Hydrazin, Borhydrid-Amin-Addukte, Natriumhydrid, Ameisensäure und andere dem Fachmann bekannte flüssige und/oder gelöste Reduktionsmittel.

Gegebenenfalls kann nach der Reduktion mit flüssigen und/oder gelösten Reduktionsmitteln das Solvens durch einen geeigneten Schritt entfernt werden. Vorzugsweise erfolgt dieser Schritt nach Durchführung der Behandlung mit einem flüssigen und/oder gelösten Reduktionsmittel. Die Entfernung des Solvens kann durch eine Trocknungs-Behandlung erfolgen. Geeignete Methoden sind unter anderem Gefriertrocknung, Sprühtrocknung, Trocknung im Hordenofen unter erhöhter Temperatur, Trocknung im Drehrohr unter erhöhter Temperatur und anderen dem Fachmann bekannte Trocknungsmethoden.

Weiterhin kann in der vorliegenden Ausführungsform auch die Behandlung des Katalysatorvorläufermateriales mit gasförmigen Reduktionsmitteln erfolgen, bevorzugt mit Wasserstoff. Diese Behandlung erfolgt durch einen thermischen Behandlungsschritt in reduzierender Atmosphäre und erfolgt bevorzugt an einem getrockneten Material. Dabei können die folgenden Gase verwendet werden: Wasserstoff, Synthesegas in variablen Zusammensetzungen, Mischungen von Kohlendioxid und Wasserstoff in variabler Zusammensetzung, Formiergas, Mischungen von Stickstoff und Wasserstoff in variabler Zusammensetzung und weitere dem Fachmann bekannte Gase und verdampfbare Flüssigkeiten, die reduzierend wirken. Auch die Gegenwart von Wasserdampf ist in die erfindungsgemäße Behandlungsatmosphäre miteingeschlossen. Die bevorzugte Temperatur des thermischen Behandlungsschrittes in reduzierender Atmosphäre liegt bevorzugt im Bereich von 20 - 1200 °C, besonders bevorzugt im Bereich von 30 - 1000 °C, ganz besonders bevorzugt im Bereich von 50 - 800 °C.

In einem nächsten Schritt (iii') zur Herstellung des korrespondierenden alternativen Katalysatorvorläufermateriales werden die geträgerten metallischen Nanopartikel mit einer Phosphorquelle kontaktiert und während dieses Schrittes oder in einem zusätzlichen Schritt, einer reduktiven Behandlung unterzogen. Erfolgt die reduktive Behandlung während des Kontaktierungsschrittes, so schließt sich ein Trocknungsschritt an. Erfolgt die reduktive Behandlung in einem zweiten Schritt nach Kontaktierung mit der Phosphorquelle, so geht der reduktiven Behandlung ein Trocknungsschritt voraus, der von einem thermischen Behandlungsschritt gefolgt sein kann.

Die reduktive Behandlung gem. des Schritts (iii') kann dabei mit flüssigen und/oder gelösten Reduktionsmitteln erfolgen. Beispiele für Reduktionsmittel sind Natriumborhydrid, Hydrazin, Borhydrid-Amin-Addukte, Natriumhydrid, Ameisensäure und andere dem Fachmann bekannte flüssige und/oder gelöste Reduktionsmittel. Weitere reduktive Behandlungsmittel, die hier zum Einsatz kommen können sind Phosphan, organo-Phosphorverbindungen mit Phosphor-Kohlenstoff-Bindungen, hochsiedende organische Phosphorverbindungen wie Tri-octyl-Phosphor-ether, elementarer Phosphor in der roten und weißen Modifikation, Phosphortrichlorid und andere dem Fachmann bekannte Phosphorverbindungen, die reduzierend wirken können. Zusätzlich zu solchen reduzierend wirkenden Phosphorverbindungen können alternative Reduktionsmittel in einem oder mehreren Schritten zum Einsatz kommen.

Weitere geeignete Phosphorquellen können unter anderem sein: Phosphorsäure, phosphorige Säure, Di-, Meta-, Poly-Phosphorsäure, Ammonium-Phosphate, Ester der Phosphorsäure, Alkaliphosphate, P₂O₅. Besonders bevorzugt sind die Phosphorquellen zur Herstellung des Materials in Wasser und organischen Lösungsmitteln löslich und/oder Mischungen aus Wasser und organischen Lösungsmitteln löslich, ganz besonders bevorzugt sind die Phosphorquellen in Wasser löslich.

Ebenfalls kann eine Behandlung mit gasförmigen Reduktionsmitteln erfolgen, bevorzugt mit Wasserstoff. Diese Behandlung erfolgt durch einen thermischen Behandlungsschritt in reduzierender Atmosphäre. Dabei können die folgenden Gase zur Anwendung kommen: Wasserstoff, Synthesegas in variablen Zusammensetzungen, Mischungen von Kohlendioxid und Wasserstoff in variabler Zusammensetzung, Formiergas, Mischungen von Stickstoff und Wasserstoff in variabler Zusammensetzung und weitere dem Fachmann bekannte Gase und verdampfbare Flüssigkeiten, die reduzierend wirken. Auch die Gegenwart von Wasserdampf ist in die erfindungsgemäße Behandlungsatmosphäre miteingeschlossen. Die bevorzugte Temperatur des thermischen Behandlungsschrittes in reduzierender Atmosphäre liegt bevorzugt im Bereich von 20 - 1200 °C, besonders bevorzugt Bereich von 30 - 1000 °C, ganz besonders bevorzugt im Bereich von 50 - 800 °C.

### III. Beispiele

Im nachfolgenden Teil werden die Herstellung der Katalysatoren und das erfindungsgemäße Verfahren zur Herstellung von Aldehyden und/oder Alkoholen beispielhaft beschrieben, wobei diese Beschreibung die Erfindung in keiner Weise einschränkt.

### III.1 Herstellung und Charakterisierung der Katalysatorbeispiele

Zur Illustration der Erfindung wurden die in der Tabelle 1 aufgeführten Katalysatorproben A1 - A8 hergestellt und zu katalytischen Testuntersuchungen verwendet. Alle in der Tabelle 1 aufgeführten Katalysatorbeispiele wurden mittels eines Imprägnierverfahrens hergestellt. Bei der Imprägnierung wurde der jeweilige Träger mit einer definierten Menge an Imprägnierlösung versetzt, die sowohl eine Rhodiumquelle als auch eine Phophorquelle enthielt. In Tab. 1 wurden die entsprechenden Spalten mit Rh-Quelle und P-Quelle gekennzeichnet. Die Menge an Imprägnierlösung wurde so gewählt, dass das Porenvolumen des Trägers vollständig mit Imprägnierlösung befüllt wurde. Das Porenvolumen der einzelnen Träger wurde zuvor anhand der Aufnahmekapazität von Wasser bestimmt. Die Stoffmenge an der jeweils zugesetzten Rhodium- und Phosphorquelle wurde in Bezug auf die Stoffmenge an Rhodium ermittelt. Bei allen Katalysatorbeispielen wurden die zugeführten Mengen so gewählt, dass die Stoffmenge an Rhodium in Bezug auf die Trägermasse bei 0,5 mmol/g lag. Dies entspricht ein Rhodiumgehalt von 5,067 Gew.- % in Bezug auf die Gesamtmasse des Katalysators. Die Gesamtmasse ergibt sich vorliegend aus der Summe der Masse der Aktivkomponente und des Trägermaterials. Bei den in Tabelle 1 und Tabelle 2 vorgeführten Trägern handelt es sich im Fall von SiC um Siliziumcarbid der Firma SICAT (Io UHP SiC Extrudate mit einer spezifischen Oberfläche von 25 m²/g); im Fall von SiO₂ um Siliziumdioxid der Firma Fuji Silysia Chemical LTD (Cariact-Q20C mit einer spezifischen Oberfläche von 140 m²/g).und im Fall von Aktivkohle um dampfaktivierten Aktivkohle der Firma Cabot (Norit Elorit mit einer spezifischen Oberfläche von 700 m2/g). Vor der Anwendung werden die Träger zerkleinert und gesiebt. Die verwendete Fraktion hat eine Korngröße von 125-160 µm.
Das Rhodium wird als Rhodium(III)nitrat-Lösung ((Umicore 1,127mol/L Rh; 9,0 Gew.-% Rh) und das Phosphor als H₃PO₄-Lösung (5 mol/L) bzw. als (NH₄)₂HPO₄-Lösung (3 mol/L) eingesetzt.

**Tabelle 1 zeigt eine zusammenfassende Übersicht zu den erfindungsgemäßen Katalysatorbeispielen A1 - A8, den eingesetzten Ausgangskomponenten und die jeweils verwendeten Rhodium-zu-Phoshor-Verhältnisse.**

| Beispiel -probe | Rh-Quelle | P-Quelle | Rh/P [mol/mol] | Träger | Beladung Metall/Träger [mmol/g] |
|---|---|---|---|---|---|
| A1 | Rh(NO₃)₃ | H₃PO₄ | 2 | SiC | 0,5 |
| A2 | Rh(NO₃)₃ | H₃PO₄ | 1 | SiC | 0,5 |
| A3 | Rh(NO₃)₃ | H₃PO₄ | 2 | SiO₂ | 0,5 |
| A4 | Rh(NO₃)₃ | (NH₄)₂HPO₄ | 2 | SiO₂ | 0,5 |
| A5 | Rh(NO₃)₃ | (NH₄)₂HPO₄ | 1 | SiO₂ | 0,5 |
| A6 | Rh(NO₃)₃ | H₃PO₄ | 2 | SiO₂ | 0,5 |
| A7 | Rh(NO₃)₃ | H₃PO₄ | 1 | SiO₂ | 0,5 |
| A8 | Rh(NO₃)₃ | H₃PO₄ | 0,5 | SiO₂ | 0,5 |

Zunächst wird die Herstellung des erfindungsgemäßen Katalysatorbeispiels A1 beschrieben. Als Träger werden 5 g eines Siliziumcarbids (Io UHP SiC Extrudates von der Firma SiCat; diese Extrudate von der Firma SiCat wiesen eine spezifische Oberfläche von 25 m²/g auf) eingesetzt, dass eine Porenvolumen 0,999 mL/g aufweist. Der hier eingesetzte Träger liegt in pulverförmiger Form vor und hat eine Korngröße im Bereich von 125-160 µm. Die entsprechende Korngrößenfraktion wurde über eine Siebung des Ausgangsmaterials erhalten. Der Träger wird auf dem Boden einer Porzellanschale verteilt und diese auf einen Rüttler befestigt.

Es werden 4,995 mL einer Imprägnierlösung hergestellt. Hierzu werden 2,218 mL (2,5 mmoL Rh) einer Rhodium(III)nitrat-Lösung (Umicore 1,127 mol/L Rh; 9,0 Gew.-% Rh), 0,25 mL (1,25 mmol P) einer 5-molaren H₃PO₄-Lösung und 2,527 mL entionisiertem Wasser vermischt. Die Imprägnierlösung wird der mit Träger befüllten Porzellanschale mittels Pipette zugeführt, während die Porzellanschale in horizontaler Richtung mit einer Rüttelgeschwindigkeit von 30 Hz und einer Amplitude von 1 cm bewegt wird. Nach Abschluss der Zugabe der Imprägnierlösung wird der mit Imprägnierlösung versetzte Träger zur Homogenisierung beziehungsweise zur Unterstützung des Trocknungsvorgangs gleichmäßig in der Porzellanschale verteilt. Zur Trocknung wird die Porzellanschale mit dem imprägnierten Träger für 16 h bei 80 °C gelagert. Anschließend wird der getrocknete Träger einer reduktiven Behandlung ausgesetzt. Hierzu wird das nach der Trocknung erhaltene Produkt (etwa 5 mL Pulver) in ein mit einer Fritte versehenes Quarzglasrohr gefüllt. Das Rohr, das eine Länge von 90 cm, einen Innendurchmesser von 13,2 mm und eine Fritte mit der Porengröße P0 hat, wird in einer Halterung eines Ofens positioniert. Die in dem Rohr befindliche Probe wird mit einem Formiergas (5 Vol.-% H₂ in N₂) beaufschlagt, das mit einer Strömungsgeschwindigkeit von 0,2 mL/min von oben nach unten durch das Rohr gleitet wird. Die Probe wird mit einer Aufheizrate von 1 K/min auf 250 °C erwärmt für eine Zeitdauer 6 h bei 250 °C getempert. Sowohl das Aufheizen als auch die Temperierung wird unter reduzierender Atmosphäre durchgeführt. Nach der Temperierung wird die im Rohr gelagerte Probe auf 20 °C abgekühlt, während weiterhin Formiergas durch das Rohr geleitet wird. Zum Abschluss wird die im Rohr gelagerte Probe einer Passivierung unterzogen. Dazu wird ein sauerstoffhaltiger Gasstrom (10 % O₂ in N₂) mit einer Strömungsgeschwindigkeit von 0,5 mL/min durch das Rohr geleitet.

Die ausgebaute Probe wird einem Siebschritt unterworfen, um die Feinanteile mit einer Teilchengröße abzutrennen. Dazu wird ein Sieb der Maschenweite von 125 µm eingesetzt. Die Teilchen, die kleiner als 125 µm sind, werden verworfen.
Die erfindungsgemäßen Katalysatorbeispiele A3 - A8 wurden in analoger Weise hergestellt, wie es hier für das Katalysatorbeispiel A1 beschrieben ist, wobei jedoch Siliziumdioxid als Träger verwendet wurde. Bei der Herstellung der Katalysatorbeispiele A4 und A5 wurde eine andere Phosphorquelle verwendet.

Die Katalysatorbeispiele VB1 - VB18 wurden als Vergleichsbeispiele in analoger Weise hergestellt, wobei jedoch andere Metallquellen als das Rhodiumnitrat und auch andere Träger verwendet wurden. Die Vergleichsbeispiele liegen nicht im Bereich der beanspruchten Erfindung, da die Katalysatoren frei von Rh oder Co sind. Als Iridiumquelle wurde eine Lösung von Iridium(III)chlorid (1 mol/l) eingesetzt. Für die Lösung wurden 17,69 g IrCl₃xH₂O (Alfa Aesar, 54,34 Gew.-% Metallgehalt) in vollentsatzten Wasser gelöst und die Lösung und auf 50 ml mit deionisiertes Wasser aufgefüllt. Als Palladiumquelle eine wurde Palladium(II)nitrat-Lösung (Umicore, 2,93 mol/l, 1,614 g/ml, 19,3 Gew.-% Metallgehalt), als Platinquelle eine Platin(II)nitrat-Lösung (Umicore, 1,753 g/ml, 30,63 Gew.-% Metallgehalt) und als Rutheniumquelle eine Rutheniumnitrosylnitrat-Lösung (Umicore, 10,46 Gew.-% Metallgehalt) eingesetzt. Bei den Trägern hadelt es sich im Fall von SiO₂ um Siliziumdioxid der Firma Fuji Silysia Chemical LTD (Cariact-Q20C mit einer spezifischen Oberfläche von 140 m²/g) und im Fall von Aktivkohle um dampfaktivierten Aktivkohle der Firma Cabot (Norit Elorit mit einer spezifischen Oberfläche von 700 m²/g). Des Weiteren unterscheidet sich auch die Form der reduktiven Behandlung. Für diese wurde 1 mL des getrockneten Vorprodukts und in einem Porzellanschiffchen (80x13x9 mm) überführt und in einem Quarzrohr mit einem Durchmesser von 40 mm und eine Länge von 1450 mm als Haltevorrichtung platziert. Das Rohr wird in einem Ofen horizontal gelegt und mit Formiergas beaufschlagt, das mit einer Strömungsgeschwindigkeit von 0,5 mL/min durch das Rohr geleitet wird. Die Temperatur wird mit einer Aufheizrate von 1 K/min angefahren und für 6 h gehalten. Nach der Temperierung wird die im Rohr gelagerte Probe auf 20°C abgekühlt, während weiter-hin Formiergas durch das Rohr geleitet wird. Zum Abschluss findet eine Passivierung analog zum anderen Prozess.

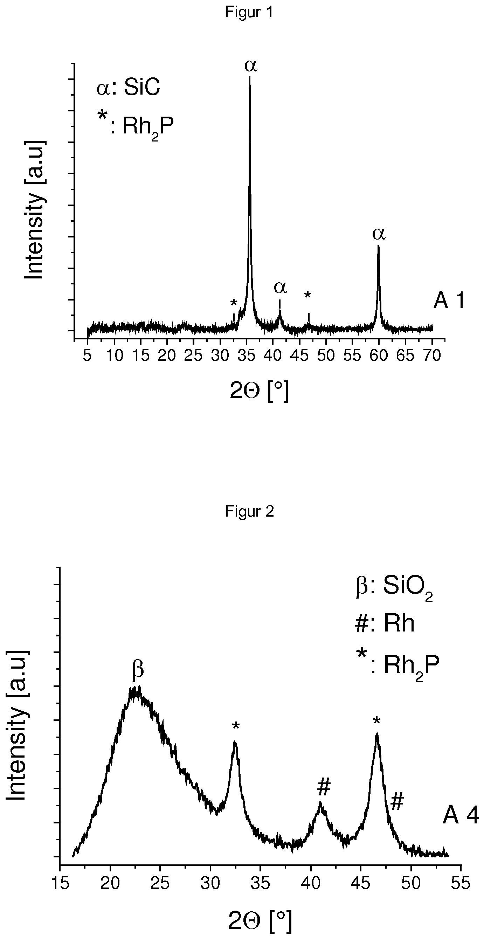

Sämtliche Proben wurden mittels XRD, TEM und XPS charakterisiert. Beispielhaft für die XRD-Analysen sind die XRD-Daten in den Figuren 1 bis 6 gezeigt.

**Tabelle 2 zeigt eine zusammenfassende Übersicht zu den Katalysatorbeispielen VB1 -VB18 (d.h. den Vergleichsbeispielen), den eingesetzten Ausgangskomponenten und Träger und die jeweils verwendeten Metall-zu-Phosphor-Verhältnisse.**

| Proben-Nummer | Zugesetztes Metallsalz für Aktivkomponente | Metall-zu-Phosphor [mol/mol] | Träger | Thermische Behandlung [°C] |
|---|---|---|---|---|
| VB1 | IrCl₃ | 0,5 | SiO₂ | 600 |
| VB2 | IrCl₃ | 1,0 | SiO₂ | 600 |
| VB3 | IrCl₃ | 2,0 | SiO₂ | 600 |
| VB4 | IrCl₃ | 0,5 | Carbon | 600 |
| VB5 | IrCl₃ | 1,0 | Carbon | 600 |
| VB6 | IrCl₃ | 2,0 | Carbon | 600 |
| VB7 | Pd(NO₃)₂ | 0,3 | SiO₂ | 700 |
| VB8 | Pd(NO₃)₂ | 1,0 | SiO₂ | 700 |
| VB9 | Pd(NO₃)₂ | 3,0 | SiO₂ | 700 |
| VB10 | Pd(NO₃)₂ | 0,3 | Carbon | 700 |
| VB11 | Pd(NO₃)₂ | 1,0 | Carbon | 700 |
| VB12 | Pd(NO₃)₂ | 3,0 | Carbon | 700 |
| VB13 | Pt(NO₃)₂ | 0,25 | SiO₂ | 550 |
| VB14 | Pt(NO₃)₂ | 1,0 | SiO₂ | 550 |
| VB15 | Pt(NO₃)₂ | 2,5 | SiO₂ | 550 |
| VB16 | Pt(NO₃)₂ | 0,25 | Carbon | 550 |
| VB17 | Pt(NO₃)₂ | 1,0 | Carbon | 550 |
| VB18 | Pt(NO₃)₂ | 2,5 | Carbon | 550 |

### III.2 Katalytische Testuntersuchungen

Um das erfindungsgemäße Verfahren zu illustrieren, wurden die Katalysatoren der Beispiele A1 - A8 (d.h. der erfindungsgemäßen Katalysatoren) und B1 - B24 (der Vergleichsbeispiele) zur Hydroformylierung von Ethen eingesetzt. Die Untersuchungen wurden mittels einer Laborreaktionsapparatur durchgeführt, die mit sechzehn parallel angeordneten Reaktoren ausgestattet war. Es wurde jeweils eine Probenmenge von 0,5 mL der Katalysatorprobe als Festbettschüttung in einen Rohrreaktor eingebracht. Die für die Testuntersuchungen eingesetzten Katalysatoren waren zuvor gesiebt worden und es wurde die Teilchenfraktion mit Teilchen im Bereich von 125 - 160 µm verwendet. Die Reaktionen wurden in der Gasphase bei einem Druck von 50 bar und einer GHSV von 2400 h⁻¹ durgeführt. Der Edukgasstrom hatte die folgende Zusammensetzung: 1 bis 10 Vol.-% Ethen, 10 Vol.-% Wasserstoff, 70 bis 79 Vol.-% Kohlenmonoxid und 10 Vol.-% Argon. Während der Durchführung der Untersuchungen wurde die Temperatur der Katalysatorproben Schrittweise in Stufen von 10 K erhöht, um die Ausbeute und Selektivität in Abhängigkeit der jeweiligen Reaktionstemperatur zu bestimmen. Bei den Untersuchungen wurden Temperaturen verwendet, die im Bereich von 170 - 240 °C lagen.

Die Produktströme wurden mittels online-Gaschromatograph untersucht, der mit den Ausgangsleitungen der einzelnen Produktabflussleitungen verbunden war und der für die Durchführung der Analysen automatisch in vorgegebenen Zeitabständen zugeschaltet wurde.

Die Ergebnisse der katalytischen Testuntersuchungen sind in den Tabellen 3-12 dargestellt, wobei jeweils die Umsätze und die Selektivitäten der Produkte aufgeführt werden. Die in den Tabellen verwendete Abkürzung Sel. steht jeweils als Abkürzung, um die Selektivität zu kennzeichnen. In der Tabelle 12 sind die Versuchsbedingungen und Ergebnisse für den Katalysator A2 (erfindungsgemäßer Katalysator mit Rhodium) gelistet.

Alle in der Tabelle 1 aufgeführten Katalysatorbeispielproben sind erfindungsgemäße Proben, die eine katalytisch aktive Komponente beziehungsweise ein aktives Phosphid auf der Basis von Rhodium im Sinne der Erfindung aufwiesen. Alle in der Tabelle 2 aufgeführten Katalysatorproben sind Vergleichsbeispiele, die nicht Gegenstand der vorliegenden Erfindung zählen. Die in den Tabellen 1 und 2 aufgeführten Katalysatorproben wurden katalytischen Experimenten zur Insertion von Kohlenmonoxid in Gegenwart von Ethen oder Propen unterzogen und zeigten allesamt ihre gute Eignung in Verbindung mit dem erfindungsgemäßen Verfahren. Ausgewählte Ergebnisse der katalytischen Testuntersuchungen werden in den Tabellen 3 - 12 gezeigt, um die Erfindung zu illustrieren.

Beispielsweise wurden auch sehr gute Ergebnisse bei einem Verfahren erzielt, dass in Verbindung mit Rhodiumphosphid als katalytisch aktive Komponente auf einem Träger aus SiC oder SiO₂ erzielt (Tabelle 3, 4, 9, 10, 11 und 12). Das katalytische Verfahren wurde auch mit Katalysatoren durchgeführt, die mit Rhodiumphosphid als katalytisch aktive Komponetne und Kohle als Träger vorlagen. Die Leistungseigenschaften der Katalysatoren mit Rhodiumphosphid als katalytisch aktiver Komponente, die auf Kohle als Träger vorlag, waren geringfügig schwächer als diejenigen Leistungseigenschaften der entsprechenden katalytisch aktiven Komponente auf Trägern aus Siliziumcarbid beziehungsweise auf Trägern aus Siliziumdioxid. Somit war auch das eingesetzte Trägermaterial von Einfluss in Bezug auf das erfindungsgemäße Verfahren.

Bemerkenswert ist, dass bei dem erfindungsgemäßen Verfahren mit ganz bestimmten Katalysatoren mit Kohlenstoff als Träger bessere katalytische Ergebnisse erzielt wurden als mit Siliziumdioxid als Träger. Bei den Katalysatoren, die eine besonders vorteilhafte Eigenschaft in Verbindung mit Kohlenstoff als Träger zu beobachten war, war die katalytisch aktive Komponente auf der Basis von einer Verbindung mit Pt, Pd oder Ir, d.h. den Katalysatorzusammensetzungen aus den Vergleichsbeispielen. Die gleichen katalytisch aktiven Komponente in den Vergleichsbeispielen (d.h. auf der Basis von Pt, Pd oder Ir) zeigten etwas geringfügigere katalytische Leistungseigenschaften in Verbindung mit dem erfindungsgemäßen Verfahren als mit SiO₂ als Träger im gleichen Verfahren.

Die Ergebnisse der katalytischen Untersuchungen für die Hydroformylierung von Ethen sind in den Tabellen 3 - 10 dargestellt, wobei jeweils die Umsätze, die Selektivitäten und die Raum-Zeit-Ausbeute der Produkte aufgeführt werden. In den Tabellen 11 und 12 sind die Ergebnisse der katalytischen Untersuchungen von Propen dargestellt.

Tabelle 3 zeigt die Ergebnisse der Untersuchungen von Katalysator A1 (Rhodiumphosphid als phosphidhaltige Aktivkomponente auf SiC als Träger) bei einem niedrigen Ethen Gehalt. Wird der Ethen Gehalt verzehnfacht (10 Vol.-%) so erzielt Katalysator A1 ähnliche Raumzeitausbeuten wie Katalysator A3 (Rhodiumphosphid als phosphidhaltige Aktivkomponente auf SiO₂), für den die Ergebnisse der katalytischen Tests in Tabelle 4.

Die Tabellen 4 bis 8 zeigen die Ergebnisse der katalytischen Testuntersuchungen verschiedener Metallphosphide. Ein Vergleich der experimentellen Daten ergibt, dass die Katalysatorproben aus Vergleichsbeispiel VB4 (Iridiumphosphid als katalytisch aktive Komponente auf Kohlenstoff) und VB11 (Palladiumphosphid als katalytisch aktive Komponente auf Kohlenstoff) sehr hohe Ethen Umsatzwerte erreichen, dabei aber nur mäßige Selektivitätswerte erzielen. Der Großteil des Ethens wird zu Ethan hydriert. Die Katalysatorproben Vergleichsbeispiel VB18 (Platinphosphid als katalytisch aktive Komponente auf Kohlenstoff) und Vergleichsbeispiel VB20 (Rutheniumphosphid als katalytisch aktive Komponente auf SiO₂) zeigen deutlich niedrige Werte für den Ethen Umsatz und nur mäßige Werte für die Selektivität von Propanal. Dies zeigt, dass sich der Katalysator mit Rhodiumphosphid als katalytisch aktive Komponente (z.B. Katalysator A3) erfindungsgemäße ganz besonders gut für die Hydroformylierung eignet.

Tabelle 9 zeigt die Ergebnisse der Testuntersuchungen von Katalysator A3 (d.h. Rhodiumphosphid als katalytisch aktive Komponente auf SiO₂ als Träger), die das erfindungsgemäße Verfahren illustrieren, bei hohen Ethen Gehalt. Auffallend ist, dass der Umsatz mit der Temperatur nahezu konstant bleibt, lediglich die Selektivität von Propanal sinkt bei steigender Temperatur. Bei dieser Verfahrensführung kann eine Raumzeitausbeute von bis 420 kg/(m³x h) erreicht werden. In den Tabellen wird die Raumzeitausbeute mit STY abgekürzt. Die Raumzeitausbeute ist in der Einheit von kg m⁻³ h⁻¹ angegeben. Dieser Wert ist etwa dreimal so hoch, wie die Werte, die an der Katalysatorprobe A3 (Rhodiumphosphid als katalytisch aktive Komponente auf SiO₂ als Träger) bei niedrigem Ethen Gehalt erzielt wurden, die in der Tabelle 4 dargestellt sind.

Tabelle 10 zeigt die Ergebnisse der katalytischen Testuntersuchung des Katalysators A1 (Rhodiumphosphid als katalytisch aktive Komponente auf SiC) in der Gegenwart von Wasser. Die Versuchsbedingungen sind identisch mit der Untersuchung in Tabelle 3 (auch Katalysatorprobe A1 mit Rhodiumphosphid als katalytisch aktive Komponente auf SiC als Träger), mit dem Unterschied, dass dem Eduktstrom H₂O zugemischt wird. Durch die Zugabe von Wasser nimmt der Ethen Umsatz leicht ab und die Selektivität von Propanal steigt deutlich, was eine Steigerung der Raumzeitausbeute ergibt. Ein Vergleich zwischen die Ergebnisse der beiden Untersuchungen bei einer Temperatur von 190 °C macht das deutlich.

Tabelle 11 zeigt die Ergebnisse der katalytischen Testuntersuchung von Katalysator A3 (Rhodiumphosphid als katalytisch aktive Komponente auf SiO₂) bei der Hydroformylierung von Propen. Die Umsatzwerte und die Raumzeitausbeute sind wesentlich niedriger als bei der Hydroformylierung von Ethen, es entstehen aber keine Nebenprodukte.

Tabelle 12 zeigt die Ergebnisse der katalytischen Testuntersuchung von Katalysator A2 (Rhodiumphosphid als katalytisch aktive Komponente auf Siliziumcarbid) bei der Hydroformylierung von Propen in der Gegenwart von Wasser bei einer Temperatur von 210 °C (die der Erfindung entsprechen). Katalysator A2 (Rhodiumphosphid katalytisch aktive Komponente auf Siliziumcarbid) erzielt, ohne die Zugabe von Wasser, ähnliche aber schlechtere Ergebnisse als der erfindungsgemäße Katalysator A3 in Tabelle 11. Durch die Zugabe von Wasser ändern sich die Selektivität Werte kaum, aber der Umsatz erhöht sich was zu einer deutlichen Erhöhung der Raumzeitausbeute führt.

### Charakterisierungsmethoden

- Die XRD-Analysen (Figur 1) wurde mit einem D8 Advance Serie 2 von der Firma Bruker/AXS unter Verwendung von CuK-alpha-Quelle (mit einer Wellenlänge von 0,154 nm bei 40 kV und 40 mA) durchgeführt. Die Messungen erfolgten über den Messbereich: 5 - 70 ° (2Theta), 0.02° Schritten mit 4,8 Sekunden/Schritt. Alle weiteren XRD Analysen wurden mit einem D8 Discover von der Firma Bruker unter Verwendung von CuK-alpha -Quelle durchgeführt Die Messungen erfolgten über den Messbereich 16 bis 53.5° (2Theta), 0.02° Schritte.
- Die TEM-Aufnahmen wurden mit einem Gerät Tecnai G2-F20ST der Firma FEI Company aufgenommen.
- Die BET-Messungen wurden mit einem Gerät der Firma Micromeritics durchgeführt. Die XPS-Messungen zur Bestimmung der chemischen Zusammensetzung wurden mit einem Gerät der Firma UV aufgenommen. Als online-Gaschromatograph wurde ein Agilent GC verwendet, der für die Auftrennung der Produktkomponenten mit einer Silica-Kapillarsäule und zur Analyse der Produktkomponenten einem Flammenionisationsdetektor ausgestattet war.
In Bezug auf die Testgase: Die katalytischen Experimente und die Behandlung der Katalysatorproben wurden mit Labor-Gasen aus Druckgaszylindern von der Firma Praxair durchgeführt. Die Reinheit des H₂- Gases lag bei 99.999 %. Die Reinheit des Ar-Gases lag bei 99,999 %, die Reinheit des Kohlenmonoxid-Gases lag bei 99,9 %.
Ethen wurde als eine Ethen/CO Gasmischung verwendet mit Konzentrationen von 10 Vol.-%, 20 Vol.-% und 30 Vol.-% eingesetzt. Für die Versuche mit hohem Ethen Gehalt wurde Ethen mit einer Reinheit von 99.9 % verwendet. Propen wurde ebenfalls als eine Propen/CO Gasmischung mit einer Konzentration von 5 Vol.-% eingesetzt. Die Analyse des Produktstroms erfolgte über einen Gaschromatograph GC 6890N von der Firma Agilent, ausgestattet mit einer Säule DB 1 (60m x 0,32mm x 3µm) und einem FID als Detektor.

**Tabelle 3 zeigt die Ergebnisse der Untersuchung zur Hydroformylierung von Ethen von der erfindungsgemäßen Probe A1 bzw. gemäß dem erfindungsgemäßen Verfahrens (1 Vol.-% Ethen, 79 Vol.-% CO, 10 Vol.-% H₂ und 10 Vol.-% Ar, GHSV = 2400 h⁻¹).**

| Temp. [C] | Umsatz Ethen [%] | Sel. Propanal | Sel. Ethan [%] | Sel. ProH [%] | Sel. BuOH [%] | Sel. PeOH [%] | Sel. CH4 [%] | Sel. C8+ [%] | STY Propanal [kg*m⁻³h⁻¹] |
|---|---|---|---|---|---|---|---|---|---|
| 170 | 36,3 | 53,1 | 35,3 | 0 | 2,8 | 1,6 | 0 | 7,2 | 6,5 |
| 180 | 46,5 | 51,4 | 36,9 | 0 | 1,6 | 1,9 | 0 | 8,2 | 8,4 |
| 190 | 54,1 | 50,8 | 37,4 | 0 | 0 | 2,1 | 0,5 | 8,7 | 9,7 |
| 200 | 65,3 | 48,3 | 38,1 | 0 | 1,0 | 2,5 | 0,7 | 8,8 | 11,3 |

**Tabelle 4 zeigt die Ergebnisse der Untersuchung zur Hydroformylierung von Ethen von der Probe A3, die erfindungsgemäß sind (10 Vol.-% Ethen, 70 Vol.-% CO, 10 Vol.-% H₂ und 10 Vol.-% Ar, GHSV = 2400 h⁻¹).**

| Temp. [C] | Umsatz Ethen [%] | Sel. Propanal [%] | Sel. Ethan [%] | Sel. ProH [%] | Sel. BuOH [%] | Sel. PeOH [%] | Sel. C8+ [%] | STY Propanal [kg m⁻³h⁻¹] |
|---|---|---|---|---|---|---|---|---|
| 170 | 25,5 | 78,5 | 18,3 | 0 | 0 | 0 | 3,2 | 79,8 |
| 180 | 34,3 | 78,6 | 17,6 | 0 | 0 | 0 | 3,8 | 108,6 |
| 190 | 41,6 | 78,0 | 17,6 | 0 | 0 | 0 | 4,4 | 132,6 |
| 200 | 51,9 | 76,9 | 17,8 | 0 | 0 | 0 | 5,3 | 165,4 |

**Tabelle 5 zeigt die Ergebnisse der Untersuchung zur Hydroformylierung von Ethen von der Probe VB4, die als Vergleichsbeispiel gezeigt werden (10 Vol.-% Ethen, 70 Vol.-% CO, 10 Vol.-% H₂ und 10 Vol.-% Ar, GHSV = 2400 h⁻¹).**

| Temp. [°C] | Umsatz Ethen [%] | Sel. Propanal [%] | Sel. Ethan [%] | Sel. Propanol [%] | Sel. PeOH [%] | Sel. C6+ [%] |
|---|---|---|---|---|---|---|
| 200 | 72,37 | 23,36 | 73,17 | 2,17 | 0,41 | 0,89 |
| 210 | 75,49 | 21,32 | 75,17 | 2,33 | 0,36 | 0,82 |
| 220 | 79,35 | 19,34 | 77,02 | 2,49 | 0,36 | 0,74 |
| 230 | 81,17 | 17,51 | 78,69 | 2,67 | 0,37 | 0,68 |

**Tabelle 6 zeigt die Ergebnisse der Untersuchung zur Hydroformylierung von Ethen von der Probe VB11, die als Vergleichsbeispiel gezeigt werden (10 Vol.-% Ethen, 70 Vol.-% CO, 10 Vol.-% H₂ und 10 Vol.-% Ar, GHSV = 2400 h⁻¹).**

| Temp. [°C] | Umsatz Ethen [%] | Sel. Propanal [%] | Sel. Ethan [%] | Sel. PrOH [%] | Sel. Propen [%] | Sel. Propan [%] | Sel. C8+ [%] |
|---|---|---|---|---|---|---|---|
| 200 | 99,53 | 9,83 | 89,83 | 0,48 | 0,08 | 0 | 1,18 |
| 210 | 99,55 | 9,46 | 90,13 | 0,45 | 0,13 | 0,10 | 0,96 |
| 220 | 99,60 | 9,08 | 90,29 | 0,41 | 0,18 | 0,20 | 0,73 |
| 230 | 99,52 | 8,75 | 90,40 | 0,48 | 0,26 | 0,42 | 0,70 |

**Tabelle 7 zeigt die Ergebnisse der Untersuchung zur Hydroformylierung von Ethen von der Probe VB18, die als Vergleichsbeispiel gezeigt werden (10 Vol.-% Ethen, 70 Vol.-% CO, 10 Vol.-% H₂ und 10 Vol.-% Ar, GHSV = 2400 h⁻¹).**

| Temp. [°C] | Umsatz Ethen [%] | Sel. Propanal [%] | Sel. Ethan [%] | Sel. Propanol [%] | Sel. C8+ [%] |
|---|---|---|---|---|---|
| 200 | 2,92 | 14,27 | 62,31 | 22,23 | 1,18 |
| 210 | 6,94 | 12,19 | 61,77 | 25,08 | 0,96 |
| 220 | 10,92 | 9,54 | 61,78 | 27,96 | 0,73 |
| 230 | 16,93 | 7,15 | 62,34 | 29,81 | 0,70 |

Bei weiteren Untersuchungen zur Hydroformylierung von Ethen wurde die Zusammensetzung des Eduktgastroms geändert. Bei einer Testung wurde der Druck auf 20 bar gesenkt und die Zusammensetzung auf 40 Vol.-% Ethen, 25 Vol.-% Wasserstoff, 25 Vol.-% Kohlenstoffmonoxid und 10 Vol.-% Argon umgestellt. Durch die Drucksenkung konnte gewährleistet, dass der Ethen weiterhin im gasförmigen Zustand vorkommt. Bei einer weiteren Testung wurde dem Eduktgasstrom Wasser hinzugefügt, um Aktivität des Katalysators zu erhöhen.

**Tabelle 9 zeigt die Ergebnisse der Untersuchung zur Hydroformylierung von Ethen bei höheren Ethen Gehalt von Katalysator A3, die dem erfindungsgemäßen Verfahren entsprechen (40 Vol.-% Ethen, 25 Vol.-% CO, 25 Vol.-% H₂ und 10 Vol.-% Ar, GHSV = 2400 h⁻¹).**

| Temp. [C] | Umsatz Ethen [%] | Sel. Propanal [%] | Sel. Ethan [%] | Sel. ProH [%] | Sel. BuOH [%] | Sel. PeOH [%] | Sel. C8+ [%] | STY Propanal [kg m-3h-1] |
|---|---|---|---|---|---|---|---|---|
| 170 | 39,2 | 67,5 | 29,0 | 0,1 | 0,2 | 0 | 3,2 | 420,0 |
| 180 | 41,9 | 61,6 | 33,5 | 0,1 | 0,2 | 0,1 | 4,5 | 406,5 |
| 190 | 41,9 | 57,4 | 36,9 | 0,1 | 0,2 | 0,1 | 5,3 | 374,1 |
| 200 | 41,8 | 52,9 | 40,6 | 0,1 | 0,2 | 0,1 | 6,0 | 338,6 |

**Tabelle 10 zeigt die Ergebnisse der Untersuchung zur Hydroformylierung von Ethen von der Probe A1 in der Gegenwart von Wasser, die dem erfindungsgemäßen Verfahren entsprechen (1 Vol.-% Ethen, 69 Vol.-% CO, 10 Vol.-% H₂, 10 Vol.-% H₂O und 10 Vol.-% Ar, GHSV = 2400 h⁻¹).**

| Temp. | Umsatz Ethen [%] | Sel. Propanal [%] | Sel. Ethan [%] | Sel. ProH [%] | Sel. BuOH [%] | Sel. PeOH [%] | Sel. CH4 [%] | Sel. C8+ [%] | STY Propanal [kg m-3h-1] |
|---|---|---|---|---|---|---|---|---|---|
| 170 | 5,6 | 100 | 0 | 0 | 0 | 0 | 0 | 0 | 2,1 |
| 190 | 47,8 | 81,8 | 17,2 | 0 | 0 | 0 | 1,0 | 15,7 | 15,7 |
| 210 | 76,7 | 78,7 | 17,8 | 0 | 1,2 | 0 | 0 | 2,2 | 25,7 |
| 230 | 96,0 | 74,32 | 19,2 | 0,9 | 1,5 | 0,4 | 0 | 3,7 | 32,2 |

Eine weitere Anwendung erfolgt durch den Einsatz der Katalysatoren A1 - A8 für die Hydroformylierung von Propen. Die Untersuchungen wurden analog zu den Untersuchungen zur Hydroformylierung von Ethen durchgeführt. Die Reaktionen wurden in der Gasphase bei einem Druck von 50 bar und eine GHSV von 2400 h⁻¹ durchgeführt. Der Eduktgastrom hatte die folgende Zusammensetzung: 1 Vol.-% Propen, 79 Vol.-% CO, 10 Vol.-% H₂, 10 Vol.-% Argon. Während der Durchführung der Untersuchungen wurde die Temperatur der Katalysatorproben Schrittweise in Stufen von 10 K erhöht, um die Ausbeute und Selektivität in Abhängigkeit der jeweiligen Reaktionstemperatur zu bestimmen. Bei den Untersuchungen wurden Temperaturen verwendet, die im Bereich von 170 - 240 °C lagen.

**Tabelle 11 zeigt die Ergebnisse der Untersuchung zur Hydroformylierung von Propen von der Probe A3, die dem erfindungsgemäßen Verfahren entsprechen (1 Vol.-% Propen, 79 Vol.-% CO, 10 Vol.-% H₂, und 10 Vol.-% Ar, GHSV = 2400 h⁻¹).**

| Temp. [C] | Umsatz Propen [%] | Sel. n-Butanal [%] | Sel. iso-Butanal [%] | Sel. Butanol [%] | Sel. C8+ [%] | STY n+iso Butanal [kg m⁻³h⁻¹] |
|---|---|---|---|---|---|---|
| 170 | 3,3 | 68,3 | 31,7 | 0 | 0 | 1,7 |
| 180 | 4,7 | 67,0 | 33,0 | 0 | 0 | 2,6 |
| 190 | 6,2 | 68,2 | 31,8 | 0 | 0 | 3,5 |
| 200 | 8,0 | 67,3 | 32,7 | 0 | 0 | 4,5 |
| 210 | 10,1 | 67,9 | 32,1 | 0 | 0 | 5,6 |
| 220 | 12,3 | 68,1 | 31,9 | 0 | 0 | 6,8 |
| 230 | 14,6 | 65,1 | 31,3 | 3,6 | 0 | 7,8 |
| 240 | 17,0 | 61,0 | 30,3 | 7,1 | 1,6 | 8,7 |

Da die Ergebnisse der Hydroformylierung von Ethen zeigen, dass die Aktivität der Katalysatoren durch die Zugabe von Wasser erhöht wird, wurden Analoge Versuche zur Hydroformylierung von Propen durchgeführt.

**Tabelle 12 fasst die Versuchsbedingungen und die Ergebnisse der Hydroformylierung von Propen in der Gegenwart von Wasser von Katalysator A2.**

| | | |
|---|---|---|
| Temperatur | [°C] | 210 |
| Propen | [%] | 3,14 |
| CO | [%] | 66,86 |
| H₂ | [%] | 10 |
| H₂O | [%] | 10 |
| Ar | [%] | 10 |
| GHSV | [hrs⁻¹] | 3163 |
| Druck | [bar] | 50 |
| Umsatz Propen | [%] | 30,63 |
| Sel. n-Butanal | [%] | 68,63 |
| Sel. iso-Butanal | [%] | 25,91 |
| Sel. Butanol | [%] | 0,81 |
| Sel. C6+ | [%] | 4,92 |
| STY n+iso Butanal | [kg/m³*hrs] | 72,27 |

- Figur 1: zeigt ein Röntgendiffraktogramm der Katalysatorprobe A1, d.h. der erfindungsgemäßen Katalysatorprobe. Die mit α gekennzeichneten Reflexe lassen sich dem als Träger verwendeten Siliciumcarbid zuordnen und die mit * gekennzeichneten Reflexe lassen sich dem Rh₂P der katalytisch aktiven Komponente zuordnen.
- Figur 2: zeigt ein XRD, das am der Katalysatorprobe aus erfindungsgemäßem Beispiel A4 aufgenommen wurde. Der sehr breite Reflex bei niedrigem 2θ Winkel (20 - 30 °) kann dem amorphen SiO₂ zugeordnet werden, das als Träger eingesetzt wurde. Stärker ausgeprägte Reflexe lassen sich dem metallischen Rh oder Rh₂P zuordnen. Diese sind im Falle von metallischem Rhodium mit Raute # oder im Fall von Phosphid mit Stern * markiert.

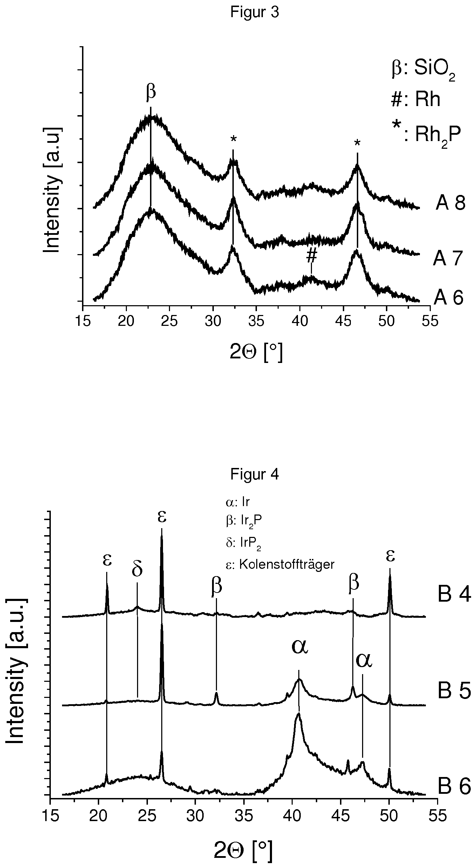

- Figur 3: zeigt ein Röntgendiffraktogramm der Katalysatorproben von den Beispiele A6 bis A8 (erfindungsgemäßen Beispielproben). Der sehr breite Reflex bei niedrigem 2θ Winkel (20-30°) kann dem amorphen Träger SiO₂ zugeordnet werden. Die stärker ausgeprägten Reflexe können dem metallischen Rh oder Rh₂P zugewiesen werden. Diese sind jeweils mit # oder mit * markiert. Der Phosphorgehalt der Katalysatorproben nimmt von A6 bis A8 zu, umgekehrt nimmt der Anteil an metallischem Rh von Probe A6 bis A8 ab. Durch den höheren Phosphorgehalt wird das gesamte Rhodium in Rhodiumphosphid umgesetzt.
- Figur 4: zeigt Röntgendiffraktogramme der Proben VB4 bis VB6 (Vergleichsbeispiele). Die einzelnen Reflexe können dem Träger () und verschiedene Ir und Ir-P Verbindungen zugewiesen werden. Diese sind im Falle von metallischem Ir mit a, im Fall von Ir₂P mit β oder im Fall von IrP₂ mit δ markiert. Analog zu Figur 3 nimmt der Phosphorgehalt von der Probe VB4 bis VB6 (innerhalb der Vergleichsbeispiele) ab. Dies wird an den Phasenverteilung der Katalysatorprobe deutlich: Die Anteile an phosphorhaltigen Verbindungen nimmt von Probe VB4 bis Probe VB6 ab.

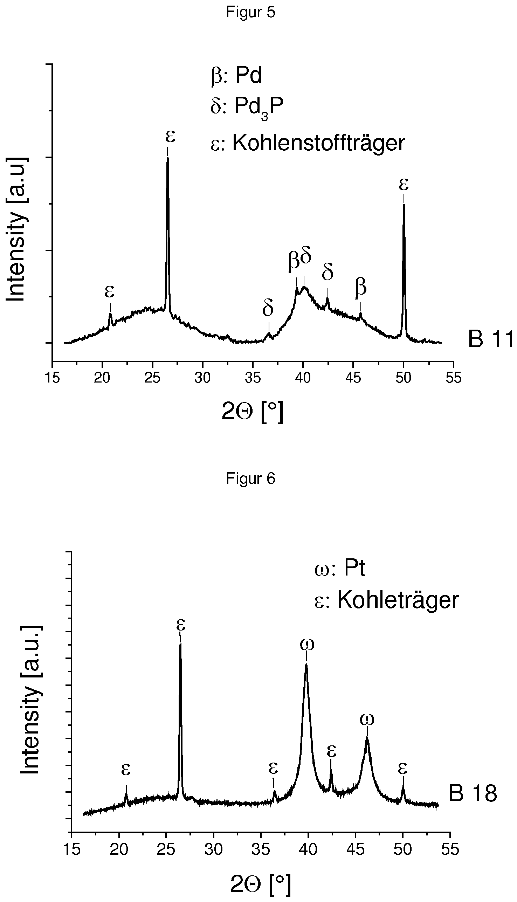

- Figur 5: zeigt ein Röntgendiffraktogramm der Probe VB11 (Vergleichsbeispiel). Die verschiedenen Reflexe können dem Träger und unterschiedliche Pd und Pd-P Verbindungen zugeordnet werden. Eine genaue Auswertung des Diffraktogramms zur Ermittlung der Teilchengröße wird durch die Breite der Reflexe limitiert.
- Figur 6: zeigt ein Röntgendiffraktogramm der Probe VB18 (Vergleichsbeispiele). Die Reflexe können dem Träger (ε) und dem metallischen Pt (ω) zugeordnet werden.

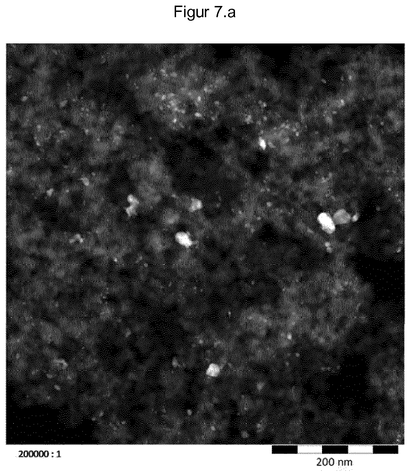

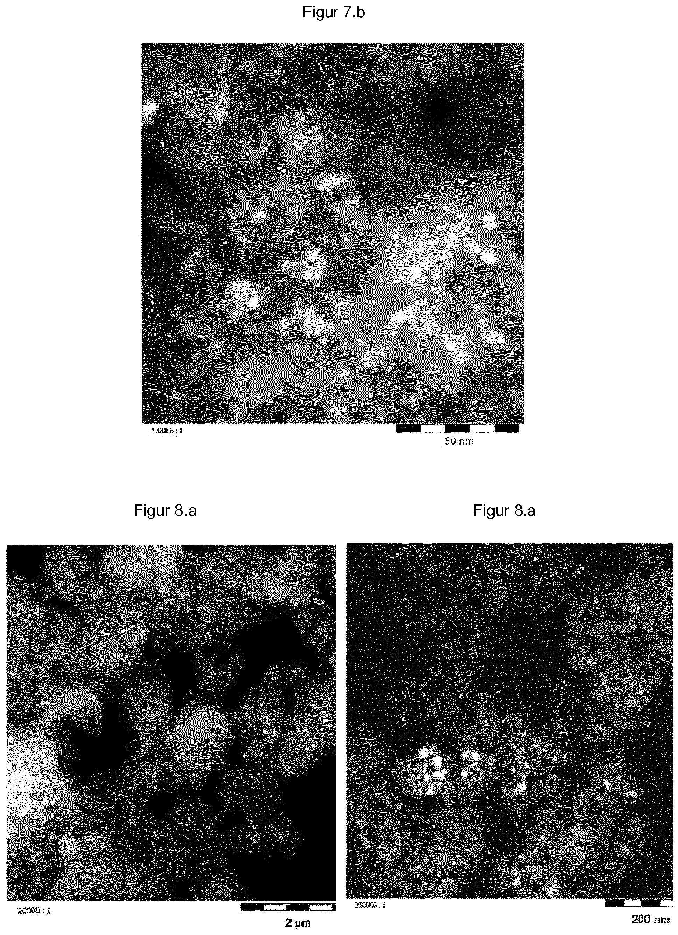

- Figur 7.a: zeigt eine TEM-Aufnahme der Probe A4 (erfindungsgemäßes Beispiel). Die hellen Bereiche zeigen die Verteilung der Rh-reichen Komponente, wie z.B.: Rh und Rh₂P. Die Aufnahme zeigt den feinteiligen, agglomerierten/aggregiertem SiO₂. Auf dem SiO₂ liegen die kristallinen, z.T. aggregierten Rh- und P-haltigen Partikel. Auf dieser Aufnahme können Partikel bzw. Agglomerate einer Größe von bis ca. 100 nm erkannt werden.
- Figur 7.b: zeigt die TEM-Aufnahme der Probe A4 (erfindungsgemäßes Beispiel) in einer höheren Auflösung. Darin sind ebenfalls die kristallinen, z.T. aggregierten Rh und P-haltigen Partikel auf dem SiO₂ zu erkennen. Die Größe der metallhaltigen Partikeln geht von ca. 3 nm bis zu ca. 50 nm. Die Annahme, dass die kleinsten Rh-haltigen Partikeln aus Einkristallen bestehen, wird durch die XRD Ergebnisse bestätigt. Mit der Halbwertsbreite der Reflexe aus dem XRD kann die Kristallitgröße mit Hilfe der Scherrer Gleichung bestimmt werden. Diese beträgt im Fall der Katalysatorprobe A4 ca. 3nm, was mit den Beobachtungen aus dem TEM übereinstimmt.

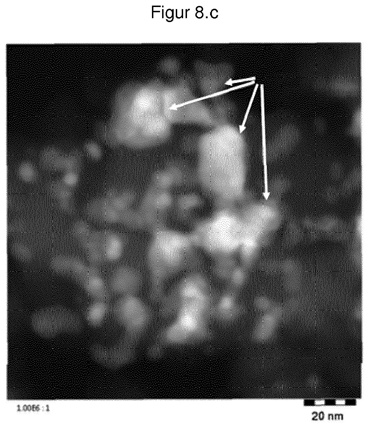

- Figur 8.a: zeigen TEM-Aufnahmen der Probe A5 (erfindungsgemäße Beispielprobe). Die hellen Bereiche zeigen die Verteilung der Rh-reichen Komponente, wie z.B.: Rh und Rh₂P. Die Partikel erscheinen feinverteilt auf dem SiO₂-Träger.
- Figur 8.b: Zeigt die TEM Aufnahme in einer höheren Auflösung. Darin sind feinverteilte so wie größere Agglomerate der Rh reiche Komponente (erfindungsgemäße Beispielprobe) zu erkennen. Dabei erreicht die Größe der Agglomerate eine etwa 20 nm.
- Figur 8.c: Zeigt die TEM Aufnahme in einer noch höheren Auflösung. Die Größe der Rhhaltigen Partikeln bzw. Agglomerate (erfindungsgemäße Beispielprobe) reicht von 3 nm bis 20 nm. Dies stimmt mit den Ergebnissen aus dem XRD über ein. Die Bestimmung der Kristallitgröße mit der Scherrer Gleichung ergibt eine Größe von ca. 2,5 nm. Die Spitzen der vier weißen Pfeile sind auf Rh-reiche Partikel gerichtet, die der katalytisch aktiven Komponente zugeordnet werden können.

## Patentansprüche

1. Verfahren zur Herstellung von C₃- bis C₁₉-Aldehyden und/oder Alkoholen durch die Hydroformylierung von Olefinen und/oder Alkinen in der Gasphase mit einem Gemisch aus Wasserstoff und Kohlenmonoxid an einem heterogenen Katalysator bei einer Temperatur von 80 - 250 °C und einem Druck von 1,5 bis 50 bar über Atmosphärendruck, in Anwesenheit eines Trägermaterials und/oder von Promotoren, **dadurch gekennzeichnet, dass** das Gemisch aus Wasserstoff und Kohlemonoxid ein H₂/CO-Verhältnis von 0,1 bis 0,9 aufweist, der Anteil an Eduktkomponente in der Form von Olefinen und/oder Alkinen im Bereich von 2 - 30 % auf Volumenbasis liegt und als katalytisch aktive Komponente des heterogenen Katalysators Rh- oder Co-Phosphid oder Gemische der beiden verwendet wird.

2. Verfahren zur Herstellung von C₃- bis C₁₉-Aldehyden und/oder Alkoholen durch die Hydroformylierung von Olefinen und/oder Alkinen in der Gasphase nach Anspruch 1, das **dadurch gekennzeichnet ist, dass** die Umsetzung der Olefine und Alkine in Gegenwart von zumindest einem Hilfsreagenz, ausgewählt aus der Gruppe Sauerstoff, Wasser, Ha-Iogenverbindungen, Ammoniak und/oder Methanol, erfolgt.

3. Verfahren zur Herstellung von C₃- bis C₁₉-Aldehyden und/oder Alkoholen durch die Hydroformylierung Umsetzung von Olefinen und/oder Alkinen in der Gasphase nach Anspruch 1 oder Anspruch 2, das **dadurch gekennzeichnet ist, dass** als katalytisch aktive Komponente des heterogenen Katalysators Rh-Phosphid verwendet wird.

4. Verfahren zur Herstellung von C₃- bis C₁₉-Aldehyden und/oder Alkoholen durch die Hydroformylierung von Olefinen und/oder Alkinen in der Gasphase nach einem der Ansprüche 1 - 3, das **dadurch gekennzeichnet ist, dass** die zur Umsetzung eingesetzten Olefine aus der Gruppe Ethen, Propen, 1-Buten, 2-Buten, Isobuten, 1-Penten, 2-Methyl-1-buten, 2-Methyl-2-buten, Cyclopenten, 1-Hexen, Cyclohexen, 1-Octen, 1-Decen, 1-Dodecen, 1-Teradecen, 1-Hexadecen, Vinylaromaten oder aus der Gruppe der sogenannten Isoolefine stammen; wobei es sich bei den Olefinen aus der Gruppe der sogenannten Isoolefine um ein oder mehrere Olefine handelt, die ausgewählt sind aus der Gruppe Isoocten, Isononen, Isodecen, Isoundecen, Isododecen, Isotetradecen, Isohexadecen.

5. Verfahren zur Herstellung von C₃- bis C₁₉-Aldehyden und/oder Alkoholen durch die Hydroformylierung von Olefinen und/oder Alkinen in der Gasphase mit einem Gemisch aus Wasserstoff und Kohlen-monoxid nach einem der Ansprüche 1 - 3, das **dadurch gekennzeichnet ist, dass** die eingesetzten Olefine und/oder Alkine zumindest eine funktionelle Gruppe tragen, bevorzugt ist die zumindest eine funktionelle Gruppe aus der Gruppe Carboxyl-, Aldehyd-, Hydroxy-, Epoxy-.

6. Verfahren zur Herstellung von C₃- bis C₁₉-Aldehyden und/oder Alkoholen durch die Hydroformylierung von Olefinen und/oder Alkinen in der Gasphase nach einem der Ansprüche 1 - 5, das **dadurch gekennzeichnet ist, dass** der Katalysator als Festbettkatalysator in einer gas/fest oder gas/flüssig/fest Reaktion eingesetzt wird und die BET-Oberfläche des Katalysators in einem Bereich von 1- 1000 m²/g liegt.

7. Verfahren zur Herstellung von C₃- bis C₅ -Aldehyden und/oder Alkoholen durch die Hydroformylierung von C₂- bis C₄- Olefinen und/oder Alkinen mit einem Gemisch aus Wasserstoff und Kohlenmonoxid nach einem der Ansprüche 1 - 6.

8. Verfahren zur Herstellung von C₃- bis C₁₉-Aldehyden und/oder Alkoholen durch die Hydroformylierung von Olefinen und/ oder Alkinen in der Gasphase nach einem der Ansprüche 1 - 7, wobei der Katalysator als heterogener Katalysator vorliegt, der **dadurch gekennzeichnet ist, dass** als katalytisch aktive Komponente des heterogenen Katalysators Rh- oder Co-Phosphid oder Gemische der beiden verwendet wird und der heterogene Katalysator in Anwesenheit eines Trägers vorliegt, vorzugsweise umfasst der Katalysator ein oder mehrere Promotoren.

9. Verfahren zur Herstellung von C₃- bis C₁₉-Aldehyden und/oder Alkoholen durch die Hydroformylierung von Olefinen und/ oder Alkinen in der Gasphase nach Anspruch 8, wobei der Katalysator **dadurch gekennzeichnet ist, dass** der Träger zumindest ein Material der Gruppe Carbide, kohlenstoffhaltige Trägermaterialien umfasst.

10. Verfahren zur Herstellung von C₃- bis C₁₉-Aldehyden und/oder Alkoholen in der Gasphase durch die Hydroformylierung von Olefinen und/ oder Alkinen in der Gasphase nach Anspruch 8 oder Anspruch 9, wobei der Katalysator **dadurch gekennzeichnet ist, dass** der Katalysator Partikel aufweist, die die katalytisch aktive Komponente enthalten, wobei die Größe der Partikel im Bereich von 1 - 100 nm liegt.

11. Verfahren zur Herstellung von C₃- bis C₁₉-Aldehyden und/oder Alkoholen durch die Hydroformylierung von Olefinen und/ oder Alkinen in der Gasphase nach einem der Ansprüche 8 - 10, wobei der Katalysator **dadurch gekennzeichnet ist, dass** die Partikel einen Anteil an katalytisch aktiver Komponente aufweist, der im Bereich von 10 - 100 Gew.-% liegt.

12. Verfahren zur Herstellung von C₃- bis C₁₉-Aldehyden und/oder Alkoholen durch die Hydroformylierung von Olefinen und/ oder Alkinen in der Gasphase nach einem der vorstehenden Ansprüche 8 - 11, wobei der Katalysator **dadurch gekennzeichnet ist, dass** der Katalysator eine Dotierung aufweist, wobei die Dotierung Verbindungen oder Elemente aus der Gruppe der Alkali- und Erdalkalimetalle, Si, Ge, Pb, Sb, As, S, Se, Te, F, Cl, Br, J umfasst.

13. Verfahren zur Herstellung von C₃- bis C₁₉-Aldehyden und/oder Alkoholen durch die Hydroformylierung von Olefinen und/ oder Alkinen in der Gasphase nach einem der vorstehenden Ansprüche 8 - 12, wobei der Katalysator **dadurch gekennzeichnet ist, dass** der Katalysator als Trägerkatalysator ausgestaltet vorliegt, wobei der Katalysator vorzugsweise als Trägerkatalysator ausgestaltet ist, die katalytisch aktive Komponente ist in Kontakt mit dem Träger und wird durch den Träger fixiert, der Trägerkatalysator weist einen Gewichtsanteil an katalytisch aktiver Komponente auf, der in Bezug auf die Summe aus katalytisch aktiver Komponente und Träger im Bereich von 0,01 - 99,5 % liegt.

14. Verfahren zur Herstellung eines Katalysators, das durch die folgenden Schritte gekennzeichnet ist:
(i) Vereinigung von Phosphorquelle, Quelle für Verbindung, die mit Phosphorquelle reagiert, und Träger,
(ii) thermische Behandlung,
(iii) reduzierende Behandlung mit Reduktionsmittel,
wobei die Verfahrensschritten (i), (ii), (iii) auch durch die Verfahrensschritte (i'), (iii') ersetzt werden können:
(i') Vereinigung von Träger mit der Quelle der Verbindung und/oder Verbindungen, die mit Phosphorquelle reagiert, und Überführung des Gemisches in einen Träger mit Nanopartikeln aus Metallen und/oder Metallverbindungen beziehungsweise Elementen,
(iii') reduzierende Behandlung, wobei der Träger mit Nanopartikeln vor oder während der reduzierenden Behandlung mit einer Phosphorquelle kontaktiert wird beziehungsweise die reduzierende Behandlung mit einer Phosphorquelle vorgenommen wird, wobei der Katalysator in einem Verfahren zur Herstellung von C₃- bis C₁₉-Aldehyden und/oder Alkoholen durch die Hydroformylierung von Olefinen und/ oder Alkinen in der Gasphase gemäß einem der Ansprüche 1 - 13 verwendet wird.

## Claims

1. A process for preparing C₃ to C₁₉ aldehydes and/or alcohols by the hydroformylation of olefins and/or alkynes in the gas phase with a mixture of hydrogen and carbon monoxide over a heterogeneous catalyst at a temperature of 80-250°C and a pressure of 1.5 to 50 bar above atmospheric pressure, in the presence of a support material and/or of promoters, wherein the mixture of hydrogen and carbon monoxide has a H₂/CO ratio of 0.1 to 0.9, the proportion of reactant component in the form of olefins and/or alkynes is in the range of 2-30% based on volume, and the catalytically active component used in the heterogeneous catalyst is rhodium phosphide or cobalt phosphide or mixtures of the two.

2. The process for preparing C₃ to C₁₉ aldehydes and/or alcohols by the hydroformylation of olefins and/or alkynes in the gas phase according to claim 1, wherein the olefins and alkynes are converted in the presence of at least one auxiliary reagent selected from the group of oxygen, water, halogen compounds, ammonia and/or methanol.

3. The process for preparing C₃ to C₁₉ aldehydes and/or alcohols by the hydroformylation conversion of olefins and/or alkynes in the gas phase according to claim 1 or claim 2, wherein the catalytically active component used in the heterogeneous catalyst is rhodium phosphide.

4. The process for preparing C₃ to C₁₉ aldehydes and/or alcohols by the hydroformylation of olefins and/or alkynes in the gas phase according to any of claims 1-3, wherein the olefins used for the conversion come from the group of ethene, propene, 1-butene, 2-butene, isobutene, 1-pentene, 2-methyl-1-butene, 2-methyl-2-butene, cyclopentene, 1-hexene, cyclohexene, 1-octene, 1-decene, 1-dodecene, 1-tetradecene, 1-hexadecene, vinylaromatics, or from the group of the isoolefins; where the olefins from the group of the isoolefins are one or more olefins selected from the group of isooctene, isononene, isodecene, isoundecene, isododecene, isotetradecene, isohexadecene.

5. The process for preparing C₃ to C₁₉ aldehydes and/or alcohols by the hydroformylation of olefins and/or alkynes in the gas phase with a mixture of hydrogen and carbon monoxide according to any of claims 1-3, wherein the olefins and/or alkynes used bear at least one functional group, the at least one functional group preferably being from the group of carboxyl, aldehyde, hydroxyl, epoxy.

6. The process for preparing C₃ to C₁₉ aldehydes and/or alcohols by the hydroformylation of olefins and/or alkynes in the gas phase according to any of claims 1-5, wherein the catalyst is used as a fixed bed catalyst in a gas/solid or gas/liquid/solid reaction, and the BET surface area of the catalyst is within a range of 1-1000 m²/g.

7. A process for preparing C₃ to C₅ aldehydes and/or alcohols by the hydroformylation of C₂ to C₄ olefins and/or alkynes with a mixture of hydrogen and carbon monoxide according to any of claims 1-6.

8. The process for preparing C₃ to C₁₉ aldehydes and/or alcohols by the hydroformylation of olefins and/or alkynes in the gas phase according to any of claims 1-7, where the catalyst is in the form of a heterogeneous catalyst, wherein the catalytically active component used in the heterogeneous catalyst is rhodium phosphide or cobalt phosphide or mixtures of the two, and the heterogeneous catalyst is present in the presence of a support, the catalyst preferably comprising one or more promoters.

9. The process for preparing C₃ to C₁₉ aldehydes and/or alcohols by the hydroformylation of olefins and/or alkynes in the gas phase according to claim 8, wherein the catalyst support comprises at least one material from the group of the carbides, carbonaceous support materials.

10. The process for preparing C₃ to C₁₉ aldehydes and/or alcohols in the gas phase by the hydroformylation of olefins and/or alkynes in the gas phase according to claim 8 of claim 9, wherein the catalyst has particles comprising the catalytically active component, where the size of the particles is in the range of 1-100 nm.

11. The process for preparing C₃ to C₁₉ aldehydes and/or alcohols by the hydroformylation of olefins and/or alkynes in the gas phase according to any of claims 8-10, wherein the catalyst particles have a proportion of catalytically active component in the range of 10-100% by weight.

12. The process for preparing C₃ to C₁₉ aldehydes and/or alcohols by the hydroformylation of olefins and/or alkynes in the gas phase according to any of the preceding claims 8-11, wherein the catalyst is doped, where the doping comprises compounds or elements from the group of the alkali metals and alkaline earth metals, Si, Ge, Pb, Sb, As, S, Se, Te, F, Cl, Br, I.

13. The process for preparing C₃ to C₁₉ aldehydes and/or alcohols by the hydroformylation of olefins and/or alkynes in the gas phase according to any of the preceding claims 8-12, wherein the catalyst is configured as a supported catalyst, where the catalyst is preferably configured as a supported catalyst, the catalytically active component is in contact with the support and is fixed by the support, the supported catalyst has a proportion by weight of catalytically active component relative to the sum total of catalytically active component and support in the range of 0.01-99.5%.

14. A process for producing a catalyst, which comprises the following steps:
(i) combining phosphorus source, source for compound that reacts with phosphorus source, and support,
(ii) thermal treatment,
(iii) reducing treatment with reducing agent,
where process steps (i), (ii), (iii) can also be replaced by process steps (i'), (iii') :
(i') combining support with the source of the compound and/or compounds that reacts with phosphorus, and transferring the mixture to a support comprising nanoparticles of metals and/or metal compounds or elements,
(iii') reducing treatment, where the support is contacted with nanoparticles before or during the reducing treatment with a phosphorus source, or the reducing treatment is undertaken with a phosphorus source, where the catalyst is used in a process for preparing C₃ to C₁₉ aldehydes and/or alcohols by the hydroformylation of olefins and/or alkynes in the gas phase according to any of claims 1-13.

## Revendications

1. Procédé pour la préparation d'aldéhydes et/ou d'alcools en C₃-C₁₉ par hydroformylation d'oléfines et/ou d'alcynes en phase gazeuse avec un mélange d'hydrogène et de monoxyde de carbone sur un catalyseur hétérogène à une température de 80-250°C et à une pression de 1,5 à 50 bars au-dessus de la pression atmosphérique en présence d'un matériau support et/ou de promoteurs, **caractérisé en ce que** le mélange d'hydrogène et de monoxyde de carbone présente un rapport H₂/CO de 0,1 à 0,9, la proportion de composant de départ sous forme d'oléfines et/ou d'alcynes se situe dans la plage de 2-30% sur base volumique et du phosphure de Rh ou de Co ou des mélanges des deux sont utilisés comme composant catalytiquement actif du catalyseur hétérogène.

2. Procédé pour la préparation d'aldéhydes et/ou d'alcools en C₃-C₁₉ par hydroformylation transformation d'oléfines et/ou d'alcynes en phase gazeuse selon la revendication 1, **caractérisé en ce que** la transformation des oléfines et des alcynes a lieu en présence d'au moins un réactif auxiliaire, choisi dans le groupe formé par l'oxygène, l'eau, les composés halogénés, l'ammoniac et/ou le méthanol.

3. Procédé pour la préparation d'aldéhydes et/ou d'alcools en C₃-C₁₉ par hydroformylation transformation d'oléfines et/ou d'alcynes en phase gazeuse selon la revendication 1 ou la revendication 2, **caractérisé en ce que** du phosphure de Rh est utilisé comme composant catalytiquement actif du catalyseur hétérogène.

4. Procédé pour la préparation d'aldéhydes et/ou d'alcools en C₃-C₁₉ par hydroformylation d'oléfines et/ou d'alcynes en phase gazeuse selon l'une quelconque des revendications 1-3, **caractérisé en ce que** les oléfines utilisées pour la transformation proviennent du groupe formé par l'éthène, le propène, le 1-butène, le 2-butène, l'isobutène, le 1-pentène, le 2-méthyl-1-butène, le 2-méthyl-2-butène, le cyclopentène, le 1-hexène, le cyclohexène, le 1-octène, le 1-décène, le 1-dodécène, le 1-tétradécène, le 1-hexadécène, les aromatiques de vinyle ou du groupe de ce qu'on appelle les isooléfines ; les oléfines du groupe de ce qu'on appelle les isooléfines étant une ou plusieurs oléfines qui sont choisies dans le groupe formé par l'isooctène, l'isononène, l'isodécène, l'iso-undécène, l'isododécène, l'isotétradécène, l'isohexadécène.

5. Procédé pour la préparation d'aldéhydes et/ou d'alcools en C₃-C₁₉ par hydroformylation d'oléfines et/ou d'alcynes en phase gazeuse avec un mélange d'hydrogène et de monoxyde de carbone selon l'une quelconque des revendications 1-3, **caractérisé en ce que** les oléfines et/ou les alcynes utilisées portent au moins un groupe fonctionnel, ledit au moins un groupe fonctionnel étant de préférence dans le groupe formé par carboxyle, aldéhyde, hydroxy, époxy.

6. Procédé pour la préparation d'aldéhydes et/ou d'alcools en C₃-C₁₉ par hydroformylation d'oléfines et/ou d'alcynes en phase gazeuse selon l'une quelconque des revendications 1-5, **caractérisé en ce que** le catalyseur est utilisé sous forme de catalyseur en lit fixe dans une réaction gaz/solide ou gaz/liquide/solide et la surface BET du catalyseur se situe dans une plage de 1-1000 m²/g.

7. Procédé pour la préparation d'aldéhydes et/ou d'alcools en C₃-C₅ par hydroformylation d'oléfines et/ou d'alcynes en C₂-C₄ avec un mélange d'hydrogène et de monoxyde de carbone selon l'une quelconque des revendications 1-6.

8. Procédé pour la préparation d'aldéhydes et/ou d'alcools en C₃-C₁₉ par hydroformylation d'oléfines et/ou d'alcynes en phase gazeuse selon l'une quelconque des revendications 1-7, le catalyseur se trouvant sous forme de catalyseur hétérogène, **caractérisé en ce que** du phosphure de Rh ou de Co ou des mélanges des deux sont utilisés comme composant catalytiquement actif du catalyseur hétérogène et le catalyseur hétérogène se trouve en présence d'un support, le catalyseur comprenant de préférence un ou plusieurs promoteurs.

9. Procédé pour la préparation d'aldéhydes et/ou d'alcools en C₃-C₁₉ par hydroformylation d'oléfines et/ou d'alcynes en phase gazeuse selon la revendication 8 ou la revendication 9, le catalyseur étant **caractérisé en ce que** le support comprend au moins un matériau du groupe formé par les carbures et les matériaux support contenant du carbone.

10. Procédé pour la préparation d'aldéhydes et/ou d'alcools en C₃-C₁₉ en phase gazeuse par hydroformylation d'oléfines et/ou d'alcynes en phase gazeuse selon la revendication 8, le catalyseur étant **caractérisé en ce que** le catalyseur présente des particules qui contiennent le composant catalytiquement actif, la grosseur des particules se situant dans la plage de 1-100 nm.

11. Procédé pour la préparation d'aldéhydes et/ou d'alcools en C₃-C₁₉ par hydroformylation d'oléfines et/ou d'alcynes en phase gazeuse selon l'une quelconque des revendications 8-10, le catalyseur étant **caractérisé en ce que** les particules présentent une proportion de composant catalytiquement actif qui se situe dans la plage de 10-100% en poids.

12. Procédé pour la préparation d'aldéhydes et/ou d'alcools en C₃-C₁₉ par hydroformylation d'oléfines et/ou d'alcynes en phase gazeuse selon l'une quelconque des revendications précédentes 8-11, le catalyseur étant **caractérisé en ce que** le catalyseur présente un dopage, le dopage comprenant des composés ou des éléments du groupe des métaux alcalins et alcalino-terreux, Si, Ge, Pb, Sb, As, S, Se, Te, F, Cl, Br, I.

13. Procédé pour la préparation d'aldéhydes et/ou d'alcools en C₃-C₁₉ par hydroformylation d'oléfines et/ou d'alcynes en phase gazeuse selon l'une quelconque des revendications précédentes 8-12, le catalyseur étant **caractérisé en ce que** le catalyseur se trouve sous forme conçue en tant que catalyseur supporté, le catalyseur étant de préférence conçu sous forme de catalyseur supporté, le composant catalytiquement actif est en contact avec le support et est fixé par le support, le catalyseur supporté présente une proportion pondérale de composant catalytiquement actif qui se situe dans la plage de 0,01-99,5% par rapport à la somme du composant catalytiquement actif et du support.

14. Procédé pour la préparation d'un catalyseur, **caractérisé par** les étapes suivantes :
(i) réunion d'une source de phosphore, d'une source du composé qui réagit avec la source de phosphore et du support,
(ii) traitement thermique,
(iii) traitement réducteur avec un agent de réduction,
les étapes de procédé (i), (ii), (iii) pouvant également être remplacées par les étapes de procédé (i'), (iii') :
(i') réunion du support avec la source du composé et/ou des composés qui réagit avec la source de phosphore et transfert du mélange dans un support présentant des nanoparticules de métaux et/ou de composés métalliques ou d'éléments,
(iii') traitement réducteur, le support présentant des nanoparticules étant mis en contact, avant ou pendant le traitement réducteur, avec une source de phosphore ou le traitement réducteur étant réalisé avec une source de phosphore, le catalyseur étant utilisé dans un procédé pour la préparation d'aldéhydes et/ou d'alcools en C₃-C₁₉ par hydroformylation d'oléfines et/ou d'alcynes en phase gazeuse selon l'une quelconque des revendications 1-13.
